# EUROPEAN PATENT SPECIFICATION

(11) **EP 2 972 335 B1**
(45) Date of publication and mention of the grant of the patent: **20.12.2017**
(21) Application number: 14710833.6
(22) Date of filing: 10.03.2014
(51) Int. Cl.: G01N 33/53, G01N 27/327, G01N 33/52

(54) **IMPROVEMENTS RELATING TO TEST DEVICES**
VERBESSERUNGEN VON TESTGERÄTE
AMELIORATIONS DE DISPOSITIFS D'ESSAI

(30) Priority: 11.03.2013 GB 201304348; 11.03.2013 US 201361775828 P
(43) Date of publication of application: 20.01.2016
(73) Proprietor: Suresensors Ltd, Inverness, IV1 1SG (GB)
(72) Inventor: O'REILLY, Tom Joseph, Inverness IV3 5SZ (GB)
(74) Representative: Macdonald, Kate
(86) International application number: PCT/EP2014/054600
(87) International publication number: WO 2014/139961

(56) References cited:
- GB-A- 1 245 999
- US-A1- 2012 094 371

## Description

### Field

The invention relates to test devices such as diagnostic test devices. In particular the invention relates to individually packaged test devices. The invention also relates to methods of manufacturing such individually packaged test devices and use of such individually packaged test devices.

### Background

Test devices such as diagnostic test devices for carrying out diagnostic tests on fluids e.g. body fluids are well known. Examples of such test devices (also known as test sensors) include glucose test devices for testing glucose concentration in body fluids such as blood, urine or saliva. The present invention is not limited to a particular kind of test device or a particular type of test.

Test devices may have various shapes and arrangements. Test devices may be provided in the form of test strips, for example substantially planar test strips having test components for conducting a diagnostic test mounted thereon. Test strips are typically relatively thin and of generally planar construction having two major surfaces and a side wall forming the periphery of the test strip lying between the two major surfaces. Test strips may be of rectangular shape having two long edges and two short edges forming the periphery of the test strip. The present invention finds particular application to test strips but is not limited to a particular shape of test strip.

The present invention is not limited to a specific kind of test strip but finds particular application in single use test strips such as blood glucose test strips.

Blood glucose measurement is used to diagnose and manage diabetes. Electrochemical determination of blood glucose typically involves placing a liquid sample (e.g. blood, urine saliva) onto a reaction zone of a test strip which contains a reagent mixture. The reagent mixture contains an enzyme capable of reacting directly or indirectly with glucose and typically also a redox mediator. The enzymatic chemical reaction is coupled to the mediator redox reaction to enable the electrochemical determination.

The reagent mixture is sensitive to moisture and must be kept dry during storage prior to use. A desiccated plastic vial containing 25 to 50 test strips is a common storage method. Opening a vial to extract a test strip exposes the remaining test strips to deleterious ambient conditions. The desiccant material brings the relative humidity down in the re-closed vial but repeated exposure to ambient conditions when removing test strips becomes a problem as the desiccant becomes additionally loaded with moisture on each occasion and its absorbent properties are consequently reduced. Also, test strips may be removed and carried separately from the vial to avoid carrying the vial around, thus exposing the test strips to ambient conditions for a harmful prolonged period. An old vial (having fully moisture loaded desiccant) may be re-used as a "lucky" vial by some patients providing minimal moisture absorption and effectively storing the test strips in ambient conditions.

Test strips may comprise test components such as measurement electrodes, a sample inlet port, a sample chamber (in which a test reaction can occur) and an air vent. In such test strips, sample fluid typically travels through the sample chamber by capillary action and the sample chamber dimensions (e.g. width and/or height) are selected appropriately to facilitate this.

Openings forming the sample inlet port and/or air vent may be formed in one or both of the major surfaces or in the side wall along the short or long edges of the typically rectangular test strip. Where a sample inlet port is formed in a side wall of a short edge, this is termed an "end fill" test strip. Where a sample inlet port is formed in a side wall of a long edge, this is termed a "side fill" test strip. Where a sample inlet port is formed on an upper major surface of a test strip, this is termed a "top fill" test strip.

One problem with test strips is degradation of reactants provided in test components due to contact with air and/or moisture and/or light (e.g. in air). This can provide inaccurate test results, and reduces shelf life and open packaging life of test strips.

Other problems include test strip handling such as packaging, extracting from packaging, picking up and manipulating the strip, locating the strip (or at least one end of) in a metering device (if required) and disposal of a used strip after the test.

Some of the problems of the vial are overcome by individually wrapping test strips in a sealed moisture proof foil laminate pouch (for example by flow wrapping).

Thus, packaging for test devices such as test strips may comprise flip top vials as described above or cartridges in which multiple test strips (typically from the same batch and having the same calibration code (if required)) are stored in a single flip top vial or cartridge. Test strips can be packaged in groups of 10, 20, 25, 50 etc. Alternatively, individual packaging for test strips has been provided by some manufacturers in which each test strip is placed between separate top and bottom covers sealed together to surround the periphery of the test strip to provide a moisture proof foil laminate pouch in a process known as flow wrapping. The top and bottom covers form in effect a sealed bag within which the test strip is freely movable.

Both the vial and pouch options present the user with issues relating to usability. Test strips stored in a vial can be difficult to remove one at a time as they are quite small and tend to come out in multiples. Foil pouches can be difficult to access due to the robust nature of the foil. Packaging large volumes into vials or pouches requires machines to singulate test strips on a separate line which typically run at slower rates than the other test strip production processes presenting manufacturing/resource planning difficulties.

Where individual packaging about each test strip is provided, the user faces the additional challenges with opening the packaging and extracting the test strip as well as handling and locating the test strip in the metering device. It should be remembered that elderly, infirm or diabetic patients may have trouble with fine motor skills and impaired sensitivity of the hands and fingers. Furthermore, individual packaging can produce unsightly and difficult to handle packaging waste. In some instances used strips may be reinserted into packaging for disposal. This may be difficult for users to do again requiring fine motor skills and finger sensitivity. Often used strips and packaging are disposed of separately with patients or carers having to handle contaminated used test strips.

As such there is interest in seeking to address these and other problems by identifying new packaging configurations that aim to: protect each test strip from the effects of moisture and/or improve the ease of use in accessing individual test strips, and/or reduce the cost and complexity of manufacture of such test strips.

GB1245999 ELFAST describes an integrated testing strip and package for a reagent having a removable covering leaf attached to the strip to protect at least the surface of the liquid absorbing material (containing colour change reagent), the covering leaf and the test strip being made of an impervious laminate of aluminium foil and plastic, the liquid absorbing layer being sandwiched between the strip and the covering leaf. ELFAST considers strip handling and describes an end of a test strip remote from the testing end of sufficient length so that it can conveniently be held between the fingers of a user. Such an arrangement may improve utility of strip handling for users, but also increases cost of manufacture impacting negatively on the amount of materials required per strip, the size of the manufacturing line (to cope with multiple strips of increased length), the size and cost of secondary packaging such as vials/cartridges.

US2012/0094371 ROSS describes a test strip having a support member with a cover sheet having a desiccant at one end and an optional sealing cap to seal a specimen into the compartment. U6488828 BHULLAR describes a re-closable bio-sensor attached to a test strip having a partitioned cover with a fixed end permanently bonded by adhesive to second substrate and separate adhesive that creates an initial seal around a sample site. The disclosure of BHULLAR is of a re-closable cover leaf attached to a test strip. WO2008/130920 POPOVICH describes a biosensor and methods of patterning a multiple well biosensor. US2009/0232702 WU describes a test device for detecting analyte in a liquid sample. EP1612553 ALLAN describes analyte measuring apparatus which prevents re-use of a test strip. WO97/27483 INCORVIA describes a top sheet and a bottom sheet as moisture barriers adhered to the test strip. WO2009/076266 YAO describes a test sensor having a tip portion which can be moved or removed to expose a channel to receive a fluid sample. To prevent or inhibit moisture ingress into the channel, the test sensor includes a tip portion. The problem of the base or lid allowing moisture ingress via their exposed major surfaces is not described or alluded to in YAO and YAO does not describe the use of cover leaves. WO2006/065705 FLEMING describes test strips as self-contained ampoules sealed to external elements comprising a base, a tab, and a capillary channel between the base and the tab and a break line intersecting the capillary channel. In one embodiment, a flexible strip attaches the base to tab but FLEMING does not describe or address the problem of the base allowing moisture ingress via exposed major surfaces. US2005/196318 MATUSEWICZ describes a sample collection device with a removably secured tab for retaining sample. US2013/0098939 ZELENY describes a cartridge for strips. US6180063 MARKART describes a test strip package having a plurality of test strips.

US6558528 MATZINGER describes electrochemical test strip cards that include integral desiccant. Electrochemical test strip precursors are provided having dry reagent in a reaction chamber bounded by opposing (top and bottom) electrodes, the reaction chamber being in gaseous communication with an integrated desiccant. Once singulated, a sample inlet port and air vent are provided in the side walls of the test strip along a long edge to form an "edge fill" test sensor. Top and bottom covers of aluminium foil and acrylic are provided. MATZINGER aims to make less use of packaging materials and provide a strip that, by utilising foil singulated strips containing desiccant, remains dry enough to last at least a month, the desiccant being less than 80% exhausted after 30 days. MATZINGER does not address strip handling.

WO2007/057704 LONG describes a disposable device comprising a first portion having a fluid application port, a handle portion hingedly attached to the first portion which normally lies in the plane of the first portion. Grip means arranged to facilitate manual handling by a user deflectably coupled to the first portion at a hinge portion are described. Insertion using the handle portion and removal of the test device from a metering device using a second portion deflectably coupled to the first portion at a hingeable portion are described; the sample application zone being located between the first and second portions of the device. LONG appears to address some problems associated with strip manipulation to insert and remove test strips hygienically from a metering device. LONG does not address strip packaging and manipulation challenges associated therewith. Nor does LONG address challenges of reagent degradation.

The Alere DETERMINE HIV 1 / 2 Ag/Ab Combo test available from Alere Limited, Stockport, Cheshire, UK is an example of a packaged test strip.

The problems of handling a strip and any associated packaging before and/or during and/or after a test remain, as do the problems of degradation of reagents over time due to moisture ingress.

The present invention seeks to alleviate one of more of the problems described herein.

### Statement of Invention

In a first aspect the invention provides a test device comprising: a test strip having first and second major surfaces and comprising: a proximal portion for bearing contacts for a metering device, an intermediate portion for receiving a sample comprising a test area on the first major surface, the test area comprising at least one test component; a separable distal portion separable so as to be movable with respect to at least the intermediate portion; and in which the test device further comprises: at least one removable cover leaf comprising a moisture barrier layer; the at least one removable cover leaf comprising a removable portion extending over at least part of one of the major surfaces of the test strip so as to seal the test area from moisture ingress and, the at least one removable cover leaf further comprising a non-removable portion attached to the separable distal portion of the test strip.

Optionally, the removable portion of the removable cover leaf extends over the first major surface of the test strip to seal the test area in respect of the first major surface.

Optionally, the removable portion of the removable cover leaf extends over the second major surface of the test strip so as to seal the test area in respect of the second major surface.

Optionally, a removable cover leaf extends over the first major surface of the test strip to seal the test area in respect of the first major surface and a further removable cover leaf extends over the second major surface of the test strip so as to seal the test area in respect of the second major surface. Thus one or two removable cover leaves according to the invention may be provided, each associated with one of the first and second major surfaces of the test strip.

Optionally, the separable distal portion of the test strip is rotatable with respect to the intermediate portion and/or to the proximal portion of the test strip.

Optionally, the separable distal portion of the test strip is connected to the intermediate portion and/or proximal portion of the test strip by one or more hinge portions.

Optionally, the separable distal portion comprises at least one arm lying alongside the intermediate portion of the test strip.

Optionally, the separable distal portion forms a frame about the intermediate distal portion of the test strip.

Optionally, the separable distal portion is U-shaped having two laterally spaced arms lying alongside the intermediate portion and a distal cross strut there between to form a frame about the intermediate portion.

Optionally, a hinge portion is provided between the or each arm and the proximal portion. Optionally, the separable distal portion of the test strip lies immediately adjacent the intermediate portion of the test strip.

Optionally, the size and/or shape of the periphery of the separable distal portion opposite the intermediate portion corresponds with the size and/or shape of the opposing periphery of the intermediate portion whereby these lie immediately adjacent one another.

Optionally, the intermediate portion is of narrower width than the proximal portion and forms an intermediate tip portion comprising a tip.

Optionally, the separable distal portion of the test strip forms a frame about the intermediate portion of the test strip, and the removable portion of the removable cover leaf extends over at least the intermediate portion and the non-removable portion of the removable cover leaf extends over the frame.

Optionally, the removable cover leaf extends over the second major surface of the test strip forming a first removable cover leaf on the second major surface of the test strip and a second cover leaf is provided extending over the first major surface of the test strip.

Optionally, the second cover leaf over the first major surface is completely detachable from the test strip.

Optionally, the second cover leaf comprises a removable portion extending over at least part of the first major surface of the test strip so as to seal the test area and having a non-removable portion attached to the proximal portion of the test strip.

Optionally, the second cover leaf comprises a second removable cover leaf having a removable portion extending over at least part of the first major surface of the test strip so as to seal the test area and having a non-removable portion also attached to the separable distal portion of the test strip.

Optionally, the separable distal portion is separable so as to be completely detachable from the remainder of the test strip.

Optionally, the separable distal portion is frangibly connected to the intermediate portion and/or proximal portion whereby the separable distal portion and associated removable portion of the removable cover leaf are detachable from the remainder of the test strip.

Optionally, the separable distal portion is separable by bending along a lateral axis across the test strip with respect to the remainder of the test strip and/or twisting along a longitudinal axis along the test strip with respect to the remainder of the test strip.

Optionally, the test component comprises a sample chamber extending from the intermediate portion into the separable distal portion and detachment of the separable distal portion opens and/or forms a sample inlet port.

Optionally, the test device comprises a removable portion of the removable cover leaf extending over at least part or all of the intermediate portion.

Optionally, the test device comprises a removable portion of the removable cover leaf extending over at least part or all of the proximal portion.

Optionally, the removable portion comprises a free tab for gripping.

Optionally, the removable portion of at least one removable cover leaf comprises pre-selected regions attached to the test strip surrounding at least part or all of the intermediate portion, or at least the test area, of the test strip. Optionally, the pre-selected regions of the removable portion attached to the test strip are attached in a manner so that at least part or all of the removable portion of the removable cover leaf is not attached, or is not attached to any appreciable extent, to the intermediate portion, or at least the test area, of the test strip. Optionally said pre-selected regions of attachment comprise a weld and/or seal and/or adhesive layer or other joint mechanism. Optionally, said pre-selected regions of attachment have different attachment strengths in the non-removable portion compared to the removable portion, the non-removable portion being fixedly attached to the test strip and the removable portion being removably attached.

Optionally, the non-removable portion of the removable cover leaf attached to the separable distal portion of the test strip comprises a distal portion of the first removable cover leaf. Optionally, first and/or second removable cover leaves are permanently attached to respective major surfaces of the separable distal portion. Optionally, the non-removable portion of the first removable cover leaf is permanently attached to the separable distal portion of the test strip and the removable portion is peelably attached to at least the intermediate portion of the test strip.

Optionally, the non-removable portion of at least one removable cover leaf is attached to the separable distal portion of the test strip and the attachment mechanism comprises a weld and/or seal and/or adhesive or other joint mechanism. Optionally, the attachment mechanism is permanent.

Optionally, the test strip comprises a substrate having a proximal portion, an intermediate portion and a separable distal portion. Optionally, the substrate is an integral component.

Optionally, the substrate comprises one or more lines of weakness and/or cuts delineating between the intermediate portion and the separable distal portion. Optionally, the substrate comprises one or more lines of weakness delineating between the proximal portion and the separable distal portion. Optionally, the first removable cover leaf (and/or second removable cover leaf where provided) comprises one or more lines of weakness and/or cuts delineating between the removable portion of the removable cover leaf and the remaining non-removable portion.

Optionally, the separable distal portion is rectangular or substantially rectangular. Optionally the test strip is rectangular or substantially rectangular.

Optionally, a pre-selected continuous region of attachment is provided on the removable portion of at least one removable cover leaf having an inner region of non-attachment sized and shaped to correspond to the intermediate portion.

Optionally, the removable portion of at least one removable cover leaf is rectangular or substantially rectangular. Optionally, the non-removable portion is rectangular or substantially rectangular.

Optionally, at least one removable cover leaf comprises a moisture barrier layer e.g. metal foil. Optionally, at least one removable cover leaf comprises a desiccant loaded plastic layer. Optionally, at least one removable cover leaf comprises a thermoplastic layer inwardly facing towards the test strip or desiccant loaded thermoplastic layer inwardly facing towards the test strip. Optionally, at least one removable cover leaf is a laminate comprising a plastic outer layer, a central moisture barrier layer and an inwardly facing tie-in layer.

Optionally, contacts are provided and these are not covered by a cover leaf or a removable cover leaf according to the invention.

Optionally, the test device comprises a removable cover leaf having a removable portion extending over at least part of one of the major surfaces of the test strip so as to seal the test area and a non-removable cover leaf on the other major surface of the test strip having a first non-removable portion extending over and attached to at least a part of said major surface of the test strip so as to seal the test area and having a second non-removable portion attached to the separable distal portion of the test strip.

In a second aspect, although not in accordance with the invention, there is provided a test device in which the cover leaf comprises a non-removable cover leaf having a first non-removable portion extending over and optionally attached to at least part of the major surface of the test strip so as to seal the test area and having a second non-removable portion attached to the separable distal portion of the test strip.

Optionally, the test device not of the invention comprises two of said non-removable cover leaves, one on each major surface of the test strip; optionally, at least one cover leaf is frangible to facilitate separation of the first and second non- removable portions upon removal of the separable distal portion; optionally, at least one cover leaf is piercable by the intermediate portion upon removal of the separable distal portion e.g. by rotation.

In a third aspect although not in accordance with the invention provides a test device comprising: a test strip having first and second major surfaces and a test area comprising at least one test component; a first cover leaf comprising a moisture and/or desiccant barrier layer on a first major surface; a second cover leaf comprising a moisture and/or desiccant barrier layer on a second major surface; wherein at least one of the cover leaves is at least partly removable from the test strip to expose the test area.

Optionally, one or more removable cover leaf comprises a moisture barrier layer e.g. metal foil. Optionally, one or more removable cover leaf comprises a desiccant loaded plastic layer. Optionally, one or more removable cover leaf comprises a thermoplastic layer inwardly facing towards the test strip or a desiccant loaded thermoplastic layer facing inwardly towards the test strip. Optionally, one or more removable cover leaf is a laminate comprising a plastic layer, a central moisture barrier layer and an inwardly facing tie-in layer.

Optionally, one or both cover leaves are removable, optionally, one or both are completely removable.

Optionally, the test strip comprises a separable distal portion which is removable along with one or both cover leaves.

Optionally, the separable distal portion is hingedly connected to the remainder of the test strip. Optionally, the separable distal portion is detachable from the remainder of the test strip. Optionally, the contacts are not covered by a removable cover leaf.

In a fourth aspect the invention provides a method of manufacturing a test device as described herein comprising: forming a test strip having first and second major surfaces and comprising: a proximal portion for bearing contacts; an intermediate portion for receiving a sample comprising a test area comprising at least one test component; a separable distal portion; applying a first removable cover leaf extending over at least part of one of the major surfaces of the test strip so as to seal the test area.

Optionally, the method comprises: attaching removably pre-selected regions of a removable portion of the removable cover leaf to the major surface of the test strip to form a seal about the test area; attaching non-removably a non-removable portion of the first removable cover leaf to the separable distal portion of the test strip.

Optionally, the method comprises: permanently attaching at least part of the non-removable portion of the removable cover leaf to the separable distal portion of the test strip.

Optionally, the method comprises: applying a cover leaf or a second removable cover leaf over the other major surface.

Optionally, the method comprises: attaching removably pre-selected regions of a removable portion of the second removable cover leaf to the major surface of the test strip to form a seal about the test area; attaching non-removably a non-removable portion of the second removable cover leaf to the separable distal portion of the test strip.

Optionally, the method comprises permanently attaching at least part of the non-removable portion of the second removable cover leaf to the separable distal portion of the test strip. Optionally, the method comprises providing cuts and/or lines of weakness in the test strip and/or in one or each cover leaf before and/or during and/or after assembly of the test device. Optionally, the method comprises providing any of the features of the device as described herein. In a fifth aspect the invention provides a method of use a test device as described herein comprising: a) optionally removing a portion (optionally a removable portion) of a cover leaf (optionally a removable cover leaf) to expose the test area; b) removing a separable distal portion of the test strip; c) inserting the proximal portion of the test strip into metering device; d) applying a sample to the sample inlet port, and e) obtaining a measurement reading from the metering device.

Optionally, in the method step (a) and/or (b) occurs before step (c). Optionally in the method step (a) occurs before step (b). Optionally in the method step (c) occurs before step (a) and (b). Optionally, in the method steps (a) and (b) occur substantially simultaneously.

In a sixth aspect the invention provides a test device card comprising two or more test devices as described herein having frangible connections between test devices.

Optionally, the test card comprises a row of test devices and/or comprising 2, 3, 4, 5, or 6 test devices as described herein.

Typically, in use, removal of the removable portion of the removable cover leaf from the intermediate portion exposes the test area (preferably in one example embodiment unsealing the sample inlet port and/or air vent) and separation (e.g. by movement or detachment) of the separable distal portion of the test strip facilitates access to the sample inlet port (particularly in an "end fill" test strip). Preferably, the sample inlet port is located adjacent to the separable distal portion. Optionally, the sample inlet port is located on a major surface of the test strip (e.g. on a first or upper major surface providing a "top fill" test strip). Preferably, the sample inlet port is located in a side wall along an edge of the test strip (e.g. a short edge for an "end fill" or a long edge for a "side fill" test strip).

Preferably the separable distal portion is removable by rotational displacement (e.g. bending or twisting) with respect to the proximal and intermediate portions and/or by detachment from the intermediate portion. Preferably, the separable distal portion is removed by bending about one or more hinge portions, typically out of the plane of a major surface of the test strip (for example about an axis between the short edges of the test strip or about an axis extending between the long edges of the test strip).

### Brief Description of the Invention

The following drawings illustrate embodiments of the invention by way of example only. In the following figures, like reference numerals refer to like features.
Figure 1 shows schematic, plan views, from above and below, and cross-sectional, elevation views along AA' of a test device according to a first example embodiment of the invention.
Figure 2 shows schematic, plan views, from above and below, and cross-sectional, elevation views along AA' of a test device according to a second example embodiment of the invention.
Figure 3 shows two schematic, plan views and two schematic, cross-sectional, elevation views of a test device according to a third example embodiment of the invention.
Figure 4 shows a schematic, plan view from below and two cross-sectional, elevation views of a test device according to a fourth example embodiment of the invention.
Figure 5 shows a schematic, plan view and four schematic, cross-sectional views of a test device according to a fifth example embodiment of the invention.
Figure 6 shows a perspective view from above of an example base substrate with printed layers for forming an example test strip for use in a test device according to the invention.
Figure 7 shows a perspective, exploded view from above of the base substrate and printed layers of Figure 6.
Figure 8 shows a perspective, exploded view from above of the example base substrate with printed layers, a spacer layer and a hydrophilic layer (also known as an anti-fog layer).
Figure 9 shows a perspective view from above of an example test strip comprising the base substrate with printed layers of Figure 6 and the spacer layer and hydrophilic layer of Figure 8.
Figure 10 shows a perspective view from above of an example test strip (such as that of Figure 9) with a shaped, selectively profiled adhesive layer on its upper major surface according to an example embodiment of the invention (e.g. the embodiment shown in Figure 2).
Figure 11 shows a perspective view from below (of, for example, the test strip of Figure 9) with a shaped, selectively profiled adhesive layer on its lower major surface according to an example embodiment of the invention (e.g. the embodiment shown in Figure 2).
Figure 12 shows a perspective, exploded view from above of an example test strip and first (in this case 'upper') removable cover leaf and second (in this case 'lower') removable cover leaf according to an example embodiment of the invention (e.g. the embodiment of Figures 10 and 11).
Figure 13 shows a perspective view from above of a test device comprising the test strip and first and second removable cover leaves of Figure 12 according to an example embodiment of the invention.
Figure 14 shows a cross-sectional, elevation view of the test device of Figure 13 along line BB' through a test area comprising test components of the test device according to an example embodiment of the invention.
Figure 15 shows a perspective view from above of a test device being readied for use according to an example embodiment of the invention with the upper removable cover leaf open (withdrawn from its initial position overlying upper major surface of intermediate tip portion 14) and the lower removable cover leaf in the process of being opened (withdrawn from its initial position) to expose the lower major surface of intermediate tip portion 14 of test strip 20.
Figure 16 shows a perspective view from above of an alternative test device according to an example embodiment the invention in which the separable distal portion of the test strip is detachable from the remainder of the test device.
Figure 17 shows a perspective view of the alternative test device of Figure 16 with the separable distal portion and the removable portion of the upper removable cover leaf removed, readying the test device for use (ready to receive a sample liquid).
Figure 18 shows a perspective, exploded view from above of the alternative test device of

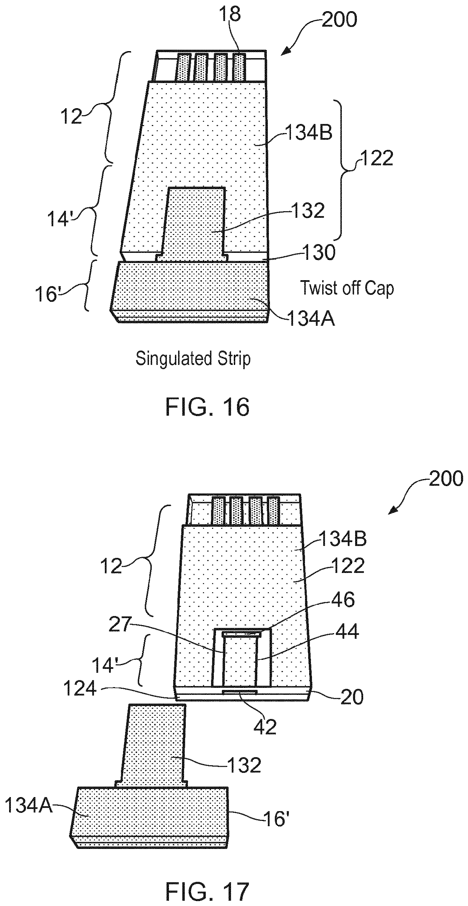

Figures 16 and 17 showing its various layers (a separate hydrophilic/antifog layer has been omitted for clarity).

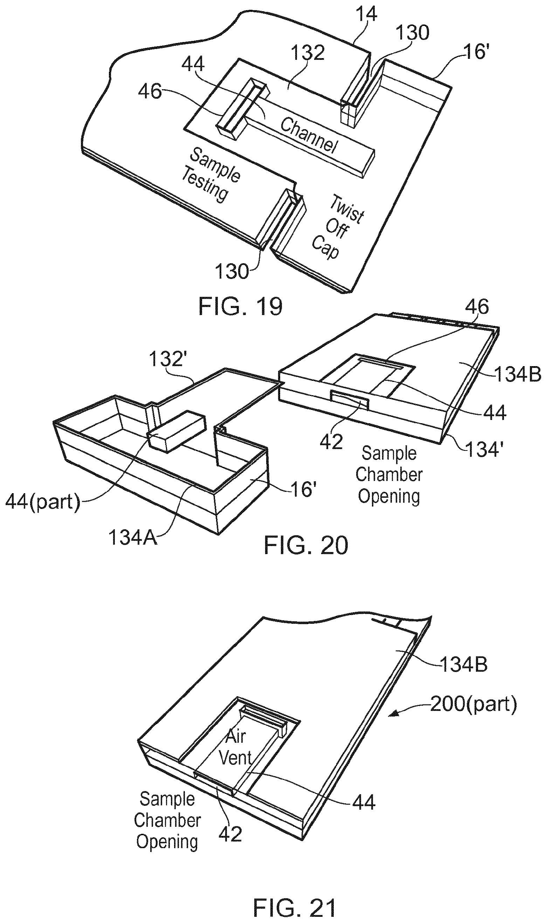

Figure 19 shows a part-cutaway, perspective view from above of the intermediate portion and separable distal portion of the alternative test device of Figure 16.
Figure 20 shows a part-cutaway, perspective view from above of the test device of Figure 16 with the separable distal portion of the test strip and removable portion of the upper removable cover leaf completely detached exposing the sample inlet port ready for use (ready to receive a sample liquid).
Figure 21 shows a perspective, close up view from above of the intermediate portion of the test device of Figure 20 ready for use.

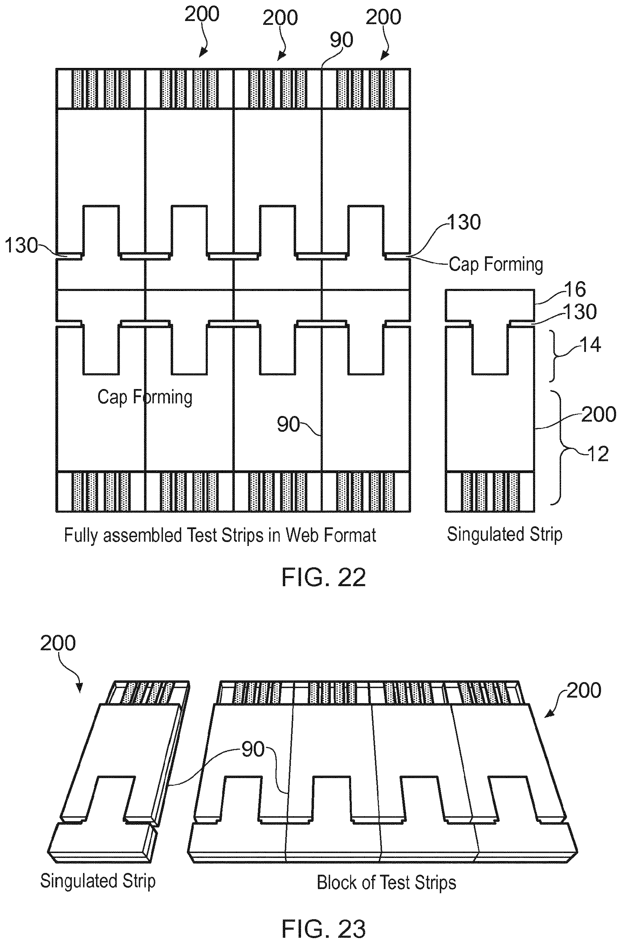

Figure 22 shows a plan view of a test device manufacturing card during manufacture comprising multiple test devices, typically in several rows, prior to singulation into individual test strips or separation into a test device user card according to a further aspect of the invention.
Figure 23 shows a perspective view of a test device user card comprising, typically a single row, of test devices (for singulation by a user prior to use) according to a further aspect of the invention.

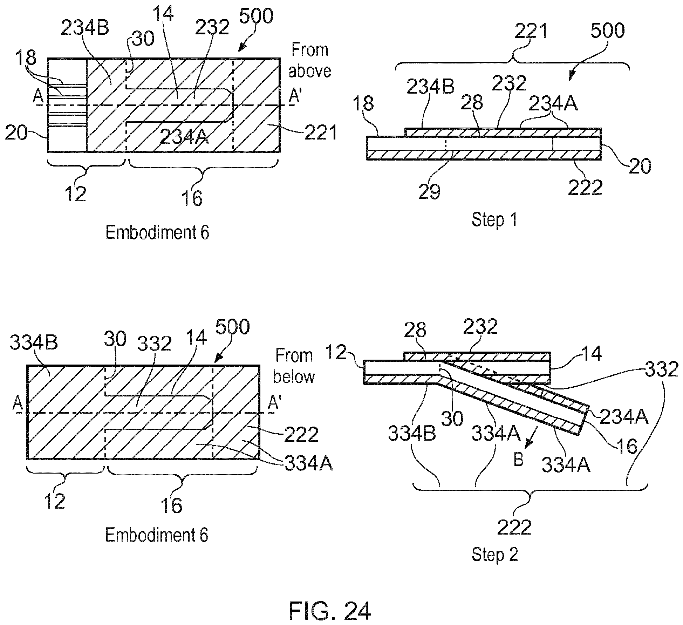

Figure 24 shows schematic plan views, from above and below, and cross-sectional elevation views of a sixth embodiment of the invention.

### Detailed Description of the Invention

Any feature of any embodiment of any aspect of the invention described herein may be combined with any other feature in any other embodiment of any aspect of the invention described herein. Further embodiments will be envisaged by those skilled in the art from the information disclosed herein and all alternative embodiments are considered to be embodiments of the invention, the invention to be limited by the appended claims.

In the previous and following descriptions and appended claims, the term 'test device' is used to refer to a combination including, but not limited to, a test strip in combination with individual packaging for the test strip in the form of one or more associated cover leaf or leaves, and the term 'test strip' is used to refer to a test sensor for conducting a test (e.g. a diagnostic test) of any suitable size or shape, although preferably of generally or substantially planar construction having upper and lower major surfaces and a side wall (preferably a relatively thin side wall) between said upper and lower major surfaces.

The present invention is not limited to a particular kind of test device or a particular type of test. The present invention finds particular application to test strips of generally or substantially planar construction having upper and lower major surfaces. The invention is not limited to a particular shape of test strip.

In the previous and following descriptions and appended claims, where the terms 'upper' and 'lower' are used with respect to the major surfaces of a test strip, test device or any portion of a test strip or test device, typically the 'upper' major surface refers to the major surface on which, in which, or in association with which, at least one test component for conducting a test (such as measurement electrodes, reagent, conductive tracks, sample chamber and hydrophilic layer) is formed and the 'lower' major surface of a test strip, test device or any portion of a test strip or test device refers to the major surface opposing the 'upper' major surface. These terms are not intended to be limiting unless the context dictates otherwise.

Similarly the terms 'first' and 'second' when used in connection with removable cover leaves and for major surfaces is not intended to be limiting unless the context dictates.

In the previous and following descriptions and appended claims, the terms 'proximal', 'intermediate' and 'distal' may be used with respect to a test device or test strip or any component of a test device or test strip e.g. a test strip, a hydrophilic layer, a spacer layer, base substrate, upper removable cover leaf or lower removable cover leaf, to indicate corresponding proximal, intermediate and distal portions of each test device, test strip or component. Nevertheless, unless indicated otherwise by the context, the terms 'proximal portion', 'intermediate (tip) portion' and 'separable distal portion' are intended to refer to the test strip component of the test device.

Test components, such as measurement electrodes, sample inlet port, sample chamber and air vent, may be provided on a major surface of a base substrate so as to provide a test area described herein as being 'on' the corresponding major surface of a test strip. It will be understood by those skilled in the art from the disclosure herein that the one or more test components if described as being 'on' a major surface of the test strip may be located upon or associated with or form part of that major surface of the test strip, for example as described herein. This is somewhat similar to patterns printed on cloth, in that the patterns are printed upon a surface of cloth but in effect form part of that surface of the cloth and are described as being 'on' the cloth but in effect form part of that surface of the cloth.

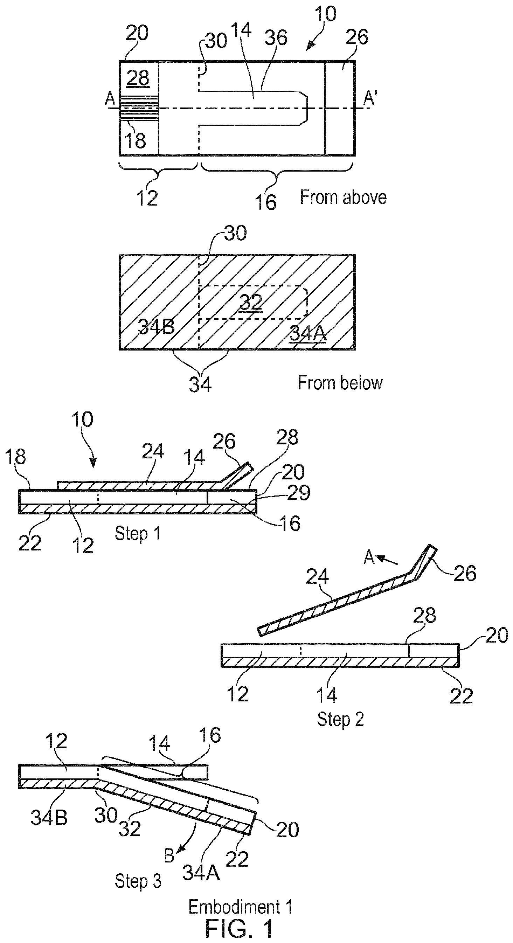

Figure 1 shows a test device 10 according to a first embodiment of the invention. Test device 10 comprises a generally planar, rectangular, test strip 20, a first (here lower) removable cover leaf 22 and a second completely detachable upper cover leaf 24 overlaying upper and lower major surfaces 28 and 29 respectively of test strip 20. Test strip 20 comprises at least one test component for conducting a test which may include one or more of, for example, a sample inlet port, a sample chamber, an air vent, conductive tracks, reagent and measurement electrodes. These test strip components are omitted to aid clarity. Thus, test strip 20 comprises an upper first major surface 28 bearing at least one and typically multiple test strip components (not shown) and an opposing lower second major surface 29.

Contacts 18, providing external electrical connection to the test strip components, are provided on an upper major surface 28 of test strip 20. In this example, contacts 18 terminate at a proximal portion of test strip 20 but alternative arrangements can be envisaged. Here, test device 10, test strip 20 and cover leaves 22 and 24 are generally rectangular, but again alternative (including but not limited to square, round, elliptical, oval) shapes can be envisaged.

Test strip 20 has a proximal portion 12, an intermediate tip portion 14 and a separable distal portion 16. Proximal portion 12 bears contacts 18. Intermediate tip portion 14 is narrower than proximal portion 12 and forms a narrower tongue extending from proximal portion 12 towards the opposing distal end of the test device. Intermediate tip portion 14 is surrounded on three sides by separable distal portion 16 when viewed from above (or below). Thus, in this example embodiment, intermediate tip portion 14 is narrower than proximal portion 12 and also narrower than separable distal portion 16. Further, in this (and other) embodiments, separable distal portion 16 is U-shaped when viewed from above having two spaced apart lateral arms, each connected directly to proximal portion 12 and a distal cross-strut connected therebetween. Separable distal portion 16 forms a U-shaped bendable frame about intermediate tip portion 14, the U-shaped bendable frame being hingedly connected to proximal portion 12.

The inner facing periphery of separable distal portion 16 matches the outer periphery of intermediate tip portion 14, preferably being of substantially the same size and/or shape. Thus, the separable distal portion surrounds and protects the side walls forming the periphery of the intermediate tip portion 14. A number of cuts 36 through test strip 20 and optionally cover leaf layers 22, 24 may be used to create and define the periphery of intermediate tip portion 14. It is preferred that the inner facing periphery of separable distal portion 16 lies immediately adjacent the periphery of the intermediate tip portion 14 about its three protruding sides. In a strip of the order of few cm long and around 0.5 cm wide, a small gap of the order of a few hundred microns to a few millimetres (up to 5 mm) may be used. The gap may be 5mm or less, although a gap of 1 or 2 mm or less is preferred. Preferably, the gap between intermediate tip portion 14 and separable distal portion 16 is the same, within manufacturing tolerances, around substantially all the periphery of the intermediate tip portion 14.

It is noted that at the distal end of intermediate tip portion 14, the corners are bevelled to create a slightly narrower distal tip end on a tip formed by intermediate tip portion 14. This may serve to guide a user as to where to place a sample.

In the example embodiment shown in Figure 1, upper first major surface 28 of test strip 20 is provided with a completely detachable upper cover leaf 24. Lower second major surface 29 of test strip 20 is provided with a first (here lower) removable cover leaf 22 which forms a removable cover leaf according to an example embodiment of the invention being removable (from its initial position) along with separable distal portion 16 of test strip 20. In more detail, lower removable cover leaf 22 comprises a central removable portion 32 overlaying intermediate tip portion 14 and a non-removable portion 34A fixedly attached to separable distal portion 16 of test strip 20. Removable portion 32 is movable (from its initial position) along with removable distal portion 16. Further, a further non-removable portion 34B is attached to proximal portion 12 of test strip 20. These will be described in more detail later.

In this and other embodiments typically the first (and/or where provided, second) removable cover leaf according to the invention will be formed of a single piece (e.g. a single piece of optionally laminated material) with, preferably, predefined removable and non-removable portions. Alternatively, the first and/or second removable cover leaves may be formed from two or more separate pieces (e.g. separate pieces of (optionally laminated) material) forming removable and non-removable portions which pieces may be joined together to form a complete removable cover leaf.

In this and other embodiments, the terms 'removable portion' and 'non-removable portion' when used in connection with a removable cover leaf according to the invention indicate the following: the portion may be moved away from its initial position (for example, overlaying a major surface of a test strip 20 and/or in line with a plane through a major surface of the test strip) - a 'removable portion'. Alternatively, the portion may not be moved from its initial position (for example, overlaying a major surface of a test strip 20 and/or in line with a plane through a major surface of the test strip) - a 'non-removable portion'.

In Figure 1, it will be noted that removable portion 32 of lower removable cover leaf 22 is removable from its initial position overlaying lower major surface of intermediate portion 14, but non-removable portion 34A remains attached to separable distal portion 16 during movement of separable distal portion 16 from its initial position in line with the plane of the remainder of the test strip. It will be noted that in this embodiment, a further non-removable portion 34B overlays a major surface of proximal portion 12 and is also not removed from its initial position with respect to that surface.

The 'removable portion' e.g. removable portion 32, of a removable cover leaf may be peelably adhered or sealed to a major surface of a test strip. One or both of the 'non-removable portions' e.g. non removable portion 34A and further non-removable portion 34B may be non-peelably (e.g. permanently) attached by adhering or welding to the test strip. Alternatively the non-removable portions (34A, 34B) may be provided with peelable or non-permanent adhesive to provide adhesion to the major surface of the test strip, but is arranged so as not to be removable (for example by not providing a free tab for gripping). Examples of adhesives, welds, seals or joints include hot welding (e.g. thermoplastic welding using polyethylene), pressure sensitive adhesives (e.g. ethylene vinyl acetate, ethyl acrylic acid), and thermosetting adhesives (e.g. acrylic, polyurethane, natural rubber).

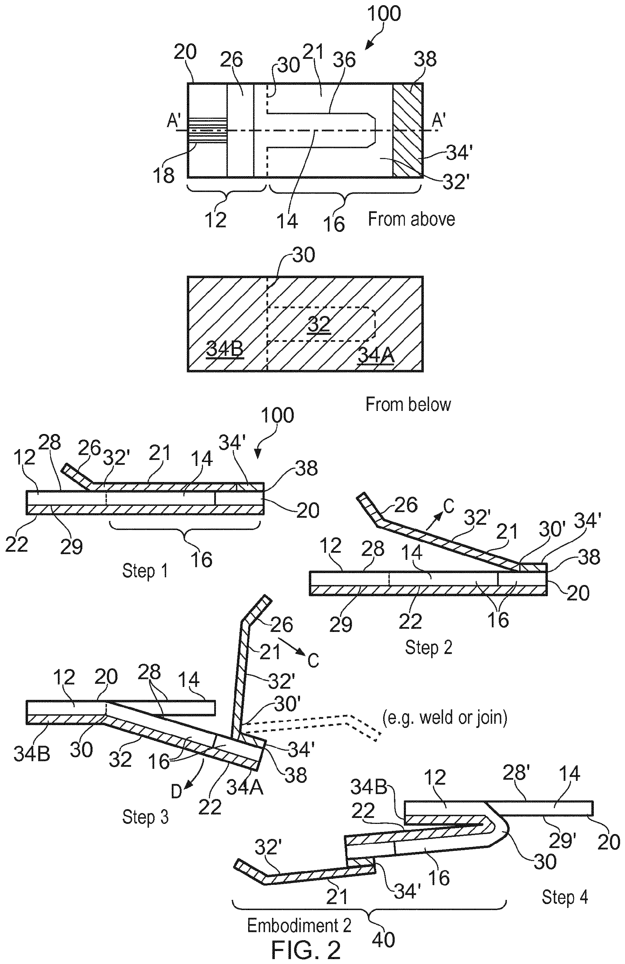

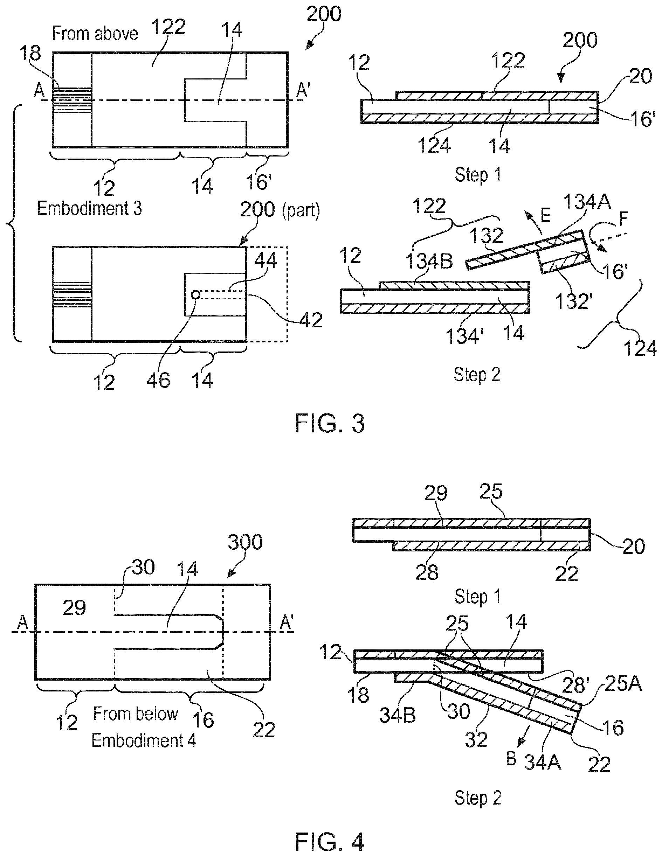

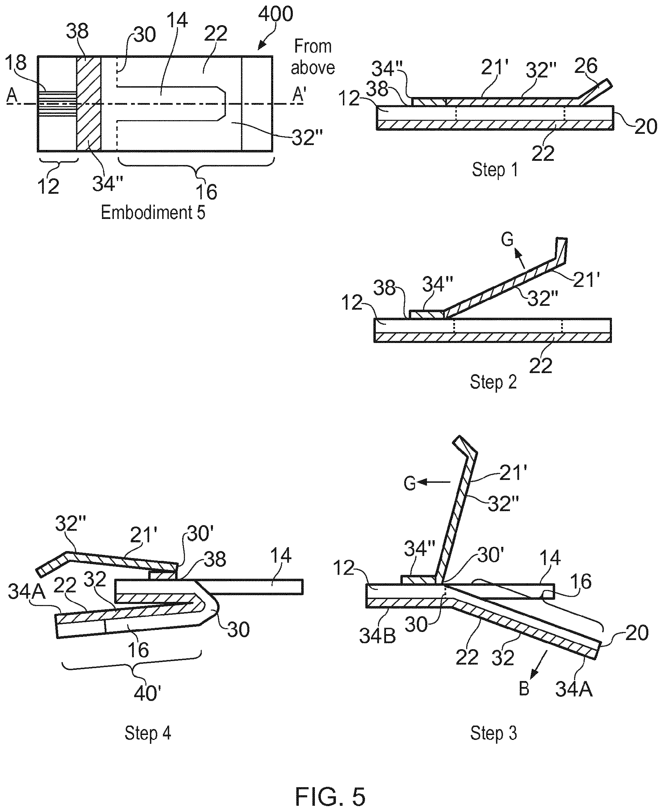

As will be seen hereinafter in various embodiments of the invention, or envisaged from the disclosure herein, the removable portion of a first removable cover leaf (and/or the removable portion of a second removable cover leaf where provided) may also be removable by complete detachment from the remainder of the test device (preferably along with the separable distal portion of the test strip, (e.g. see Figure 3) contemporaneously or subsequently) or may be removable by moving away from an initial position overlaying a major surface of a test strip without being completely detached from the remainder of the test device (e.g. see Figures 1, 2 ,4 and 5). In Figures 1, 2, 4 and 5, the removable portion 32 (32' in Figure 2, 32" in Figure 5) separates from intermediate portion 14 of the test strip and travels with associated non-removable portion 34A and separable distal portion 16 as these move for example, as these rotate out of position.

Similarly, the separable distal portion of the test strip to which the removable portion(s) of the first (and/or second) removable cover leaf is attached, optionally permanently, may be separable by complete detachment from the remainder of the test device or may be separable by moving the separable distal portion away from its initial position with respect to part or all of the remainder of the test strip 20.

In the example embodiment shown in Figure 1, movement of the separable distal portion 16 and associated removable portion 32 and non-removable portion 34A of lower removable cover leaf 22 exposes the intermediate tip portion 14 ready for use (ready to receive a sample). As will be seen later in relation to Figures 2 and 6 to 15, movement of the separable distal portion 16 and associated removable portions 32, 32' of lower and upper removable cover leaves 22, 21 exposes the intermediate tip portion 14 ready for use.

Referring now to Figure 1, an upper detachable cover leaf 24 is provided with a tab 26 free from any adhesive for taking hold of to aid removal of cover leaf 24. Typically, a peelable adhesive is used to seal selectively (in selected regions) so as to surround the periphery of intermediate tip portion 14 sealing it from above. The seal may be provided by the peelable adhesive and may extend inwardly a little over the gap formed by the cuts 36 to surround and cover the periphery of intermediate tip portion 14. This may provide additional sealing about the side walls of intermediate portion 14 (as the adhesive or thermal weld or seal may creep into the gap). Such a sealing arrangement may also be provided in the case in which an upper removable cover leaf having removable and non-removable portions (for example see Figures 2 and 5) is provided instead of detachable cover leaf 24. Upper detachable cover leaf 24 overlays most of upper major surface 28 of test strip 20, but preferably stops short over proximal portion 12 so as to expose contacts 18, the utility of which will be described later. Upper detachable cover leaf 24 may be replaced by an upper removable cover leaf (21 in Figure 2, 21' in Figure 5) having removable portions (32' in Figure 2, 32" in Figure 5) and non-removable portions, (34' in Figure 2 and 34" in Figure 5) respectively. In Figure 2, the upper removable cover leaf 21 is a removable cover leaf according to the invention having a non-removable portion fixedly attached (preferably permanently) to the separable distal portion 16 of the test strip 20. Thus the test device of the invention may be provided with one or two cover leaves having removable and non-removable portions. Preferably as in Figure 2 the non-removable portion of upper and lower cover leaves are fixed to the separable distal portion 16 to provide removable cover leaves according to the invention. In one alternative, the non -removable portion of one cover leaf is fixed to proximal portion 12 as in Figure 5. In another alternative, one removable cover leaf having a non-removable portion is fixed to the separable distal portion 16 and a detachable cover leaf such as upper detachable cover leaf 24 may also be provided as in Figure 1.

Test strip 20 comprises a substrate with printed layers as will be described later. The substrate is typically formed as an integral component of a suitable material such as plastic. Examples of plastics used include polyethylene terapthalate, polypropylene. Test strip 20 is provided with two laterally spaced hinge portions 30, optionally formed from a line of weakness or other hinge mechanism, joining proximal portion 12 and separable distal portion 16, and enabling separable distal portion 16 to form a bendable U-shaped frame (bendable about hinge portions 30) surrounding intermediate tip portion 14 having two arms lying alongside intermediate tip portion 14 and a cross-strut therebetween. Separable distal portion 16 has a non-removable portion 34A of first removable cover leaf 22 attached thereto. In this example embodiment both the intermediate tip portion 14 and the separable distal portion 16 are directly connected to the proximal portion 12. Preferably, there is no hinge portion between proximal portion 12 and intermediate tip portion 14.

Lower removable cover leaf 22 forming in this example embodiment a first removable cover leaf according to the invention, overlies at least part of lower major surface 29 of test strip 20. The lower removable cover leaf 22 is sealed in selected regions to lower major surface 29. In particular, preferably, the lower removable cover leaf is sealed so as to surround the periphery of the intermediate tip portion 14 (e.g. so as to seal the test area) as will be described in more detail later, but the seal does not extend over the intermediate tip portion 14 to any appreciable extent so this is not sealed to the removable portion 32 of the removable cover leaf 22 or is not sealed to any appreciable extent. The seal may be permanent or temporary (e.g. a thermo plastic weld or peelable adhesive). As described above the seal may extend inwardly a little over the gap formed by cuts 36 to surround and cover the periphery of the intermediate tip portion 14. This may provide additional sealing of the side walls of intermediate tip portion 14 as the adhesive of thermoplastic may creep into the gap. Examples of seal layouts (e.g. thermo weld or adhesive layouts) for use with cover leaves will be described in more detail below. Thus, typically the lower removable cover leaf 22 is not sealed to the intermediate tip portion 14, so that, the removable portion 32 of the lower removable cover leaf 22 can be removed without pulling on or bending the intermediate tip portion 14 when separable distal portion 16 and associated non-removable portion 34A of removable cover leaf 22 are rotated about hinge portions 30. As described above, non-removable portion 34A of removable cover leaf 22 may be sealed, e.g. permanently welded, permanently adhered, or peelably adhered to separable distal portion 16 and moves with it. A further non-removable portion 34B may be similarly sealed to the proximal portion 12 of the test strip.

Referring to Figure 1, in use in step 1, a test device 10 is provided comprising a test strip 20 having an upper detachable cover leaf 24 and a lower removable cover leaf 22 (here a first removable cover leaf according to the invention) mounted thereon, the test strip 20 having a proximal portion 12, an intermediate tip portion 14 and a separable distal portion 16 in the form of a U-shaped bendable frame surrounding the intermediate tip portion 14 on three sides. The removable portion 32 of the lower removable cover leaf 22 is not adhered to intermediate tip portion 14 (although non-removable portion 34A is typically adhered to the separable distal portion 16). Nevertheless, removable portion 32 extends over intermediate tip portion 14 initially but is carried away from it with the separable distal portion 16 as this rotates.

In step 2, tab 26 is gripped and pulled by user in the direction of arrow A to peelably detach detachable cover leaf 24 from test strip 20, thereby exposing (in respect of the upper major surface) a test area of test strip 20 which test area comprises at least one test component (such as sample inlet port, sample chamber and air vent) located on intermediate tip portion 14. Strictly speaking, the test components are located, typically in layers, on the upper major surface of a base substrate to form the test strip.

In step 3, separable distal portion 16 is gripped (typically by the cross strut at a distal end thereof) and rotated about hinge portion 30 relative to proximal portion 12 and relative to intermediate tip portion 14 in the direction of arrow B. As intermediate tip portion 14 is not attached to the removable cover leaf 22, the removable portion 32 of the removable cover leaf 22 initially overlying intermediate tip portion 14 simply rotates with the separable distal portion 16 (and with non-removable portion 34A) out of the plane of the remainder of the test device (proximal portion 12 and intermediate tip portion 14) thereby exposing the lower major surface of intermediate tip portion 14. Intermediate tip portion 14 therefore stands proud of the separable distal portion 16 and removable portion 32 of removable cover leaf 22 and provides easy access for a user to a sample inlet port e.g. at an end (for "end fill") or at a side (for "side fill") of intermediate tip portion 14.

Separable distal portion 16 may be bendable backwards through almost 180° about hinge portion 30 to lie alongside the proximal portion 12 of the test device to provide additional length (in effect an extended 'handle') to the test device 10 to facilitate device handling. Thus separable distal portion 16 may be used as a handle before, and/or during, and/or after sample application and sample measurement. Furthermore as separable distal portion 16 carries removable portion 32 of removable of removable cover leaf 22, replacement of separable distal portion 16 to its initial position, in the plane containing the remainder of test strip 20, re-covers the exposed intermediate tip portion 14 about its lower major surface and about its periphery.

Referring to Figure 2, a further improved embodiment of the invention is shown which works in a similar manner to Figure 1 (in respect of the lower removable cover leaf 22) having upper and lower removable cover leaves 21 and 22 according to an example embodiment of the invention, each being movable along with the separable distal portion 16. It is conceivable that each may be movable with two or more separate separable distal portions 16 of test strip 20, although this is less preferred.

In this embodiment, test device 100 comprises a test strip 20, a first (here upper) removable cover leaf 21 and a second (here lower) removable cover leaf 22. The test strip 20 is formed from an integral substrate and has three sections a proximal portion 12 (typically bearing electrical contacts 18), an intermediate tip portion 14 and a separable distal portion 16. Proximal portion 12 is optionally not completely covered by a cover leaf or is covered but not sealed to a cover leaf so electrical contacts 18 are exposed. Thus, in this and other embodiments an unused strip can be inserted (typically electrical contacts first) into a metering device before exposing test components to atmosphere. The metering device may then be usable as a handle to hold the test device 100 whilst the removable cover leaves 21 and 22 are removed by a user. Furthermore in this and other embodiments this arrangement enables the metering device to be used to conduct measurement before the removable cover leaf/leaves are removed. This pre-test measurement may be used to assess the viability of the test strip 20 and/or the degradation of the reagent and/or assess the test strip for manufacturing flaws before the test strip is used. If an error or out of scope reading is taken in a pre-test measurement, an error message can be delivered to a user before a test is conducted, reducing the possibility of an incorrect test measurement being delivered to a user and the risk of inappropriate treatment and avoiding the inconvenience of multiple wasted strips, or multiple finger sticks to obtain a sample as well as time taken conducting a wasted test.

In the example embodiment shown in Figure 2, upper major surface 28 of test strip 20 is overlaid by a first removable cover leaf 21 having a removable portion 32' including an optional tab 26 and a non-removable portion 34'. Upper non-removable portion 34' is permanently attached to a distal end of U-shaped separable distal portion 16 by a weld or seal or adhesive or other joint mechanism at seam 38. Upper removable portion 32' extends over at least the intermediate portion 14 and the arms of the U-shaped frame forming the separable distal portion 16 and may extend over proximal portion 12. Upper removable portion 32'is rotatable, in use about a hinge portion 30' in upper removable cover leaf 22. Upper non-removable portion 34' may be rectangular. Upper removable portion 32;' may be rectangular and is typically the size and shape of the intermediate portion and the separable distal portion of the test strip 20.

The lower major surface 29 of the test strip 20 is overlaid by a second removable cover leaf 22 having a removable portion 32 and a non-removable portion 34A overlaying the separable distal portion 16 of the test strip and a further non-removable portion 34B overlaying the proximal portion of the test strip. Lower removable portion 32, non-removable portion 34A and separable distal portion 16 are rotatable, in use, about two laterally spaced hinge portions 30 in test strip 20. Typically hinge portions 30 are formed by a line of weakness (such a score or partial cut or other hinge mechanism) in the substrate of test strip 20 across the join between the lateral arms of separable distal portion 16 and proximal portion 12. Alternatively, or preferably in addition, a hinge portion (not shown) such as a score, partial cut out or other hinge mechanism may be provided in lower removable cover leaf 22 in the region of hinge portions 30.

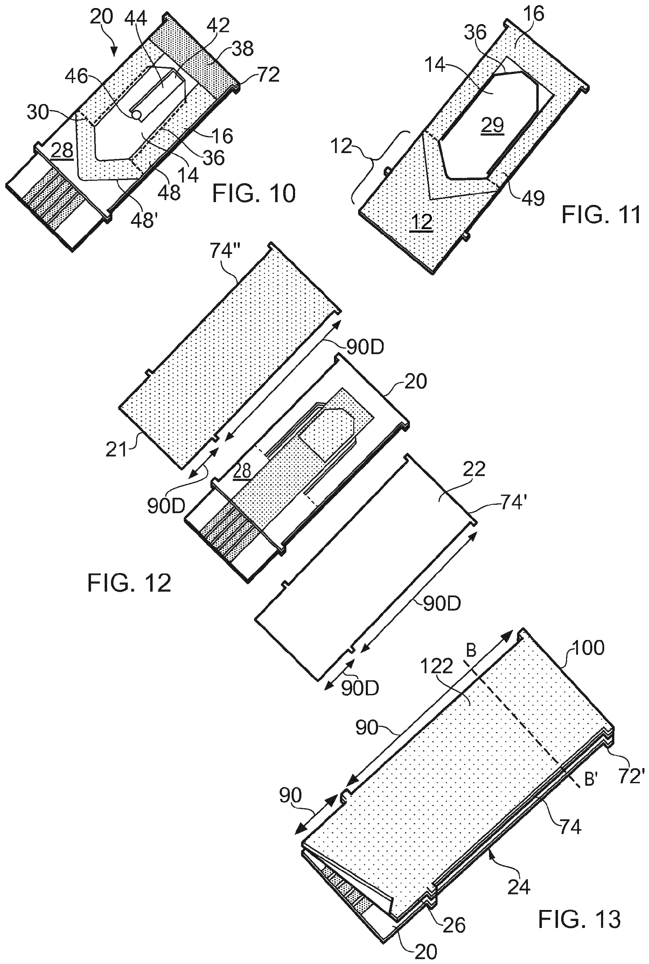

Upper removable cover leaf 21 may overlay at least around ¾ of the upper major surface 28 of test strip 20, having a non-removable portion 34' attached (preferably permanently) to a distal end of separable distal portion 16 by a (preferably permanent) seam 38. Seam 38 may form part of seal 48 (see Figure 10). Typically removable cover leaf 21 is of the same size and shape as test strip 20 in the regions it covers on test strip 20. Removable portion 32' is typically of the same size and/or shape as the test strip 20 in the regions it covers and typically it terminates in a free tab 26 that is not adhered to upper major surface 28. The remainder of removable portion 32' is selectively sealed in regions on upper surface 28 in a manner so that intermediate tip 14 is surrounded by a seal between the upper removable cover leaf 21 and the test strip 20. Thus in this embodiment, as intermediate tip 14 is generally rectangular, a generally rectangular seal slightly bigger than intermediate tip portion 14 is provided forming a continuous seal about intermediate tip portion 14. In this example the continuous seal is made up of preferably permanent seam 38 and a temporary (e.g. peelable) seal extending along the lateral arms of separable distal portion 16 (here a U-shaped frame) and across the proximal portion there between so that the intermediate tip portion is sealed from above. It is preferred that the region of the removable cover leaf 21 directly opposite intermediate tip portion 14 is completely or substantially free of adhesive so that when removable cover leaf 21 is removed, intermediate tip portion 14 is not perturbed. It may be that the seal extends over (and slightly into) the narrow gap 36 around intermediate tip portion 14, perhaps even extending slightly over intermediate tip portion 14 to provide additional sealing as described above. However it is preferred that this seal does not extend onto intermediate tip portion 14 or attach the cover to intermediate tip portion 14 to any appreciable extent.

Alternative embodiments can be envisaged in which a region of weak adhesive, or other weak seal, may be provided on intermediate tip portion 14, but this is less preferred. Similarly a profiled adhesive layer may be provided for lower removable cover leaf 22 having regions that are selectively adhered to lower major surface 29 (for example to separable distal portion 16, and the proximal portion 12) and not adhered to any appreciable extent (physically or chemically) to intermediate tip portion 14. The profiled adhesive layer may extend over the gap in the region of cuts 36 (as described above).

In the example embodiment shown in Figure 2 both upper and lower removable cover leaves 21 and 22 remain attached to separable distal portion 16 of the test strip 20 during use although these are removable having removable portions 32' and 32 which can be removed, by rotation out of the plane of the test strip. In this case, this rotation occurs about two separate longitudinally spaced axes (through hinge portions 30' and 30). The axes of rotation though hinge portions 30' and 30 lie (initially) in the plane of the test strip and substantially perpendicular to an axis through the test strip (here perpendicular to a longitudinal axis of an elongate test strip, extending from a proximal end (bearing contacts) of the test strip to a distal end (near or adjacent a sample inlet port)).

The removable portion 32' of the upper removable cover leaf 21 (overlaying the intermediate tip portion 14 and the arms of the U-shaped frame forming the separable distal portion 16) rotates about the axis through hinge portions 30' in the direction of arrow C (away from the proximal portion 12), whereas the removable portion 32 of the lower removable cover leaf 22 (and the non-removable portion 34A overlaying the intermediate tip portion 14) rotates through hinge portion 30 in the direction of arrow D (towards the proximal portion 12). This arrangement of spaced, but substantially parallel axes of rotation, one for each removable portion of the upper and lower removable cover leaves 21, 22, facilitates the provision of a doubly extended handle 40 comprising separable distal portion 16 and removable portion 32 (and non-removable portion 34A) of lower removable cover leaf 22 and removable portion 32'of upper removable cover leaf 21. This extended handle 40 greatly extends the usability of the test strip without requiring additional materials.

In step 1 of Figure 2 a test device 100 as shown is provided. In step 2 tab 26 is pulled in the direction of arrow C rotating removable portion 32' about hinge portion 30' and removing it from upper surface 28 of test strip 20, removing the seal positioned around (and the cover positioned over) intermediate tip portion 14. At some point prior to steps 2 and/or 3 and/or 4 or after step 4, the test device 100 may be inserted into a metering device and a pre-test conducted e.g. to identify if the strip is in date and/or if the strip is the correct test strip for the meter. If this occurs before step 2 and/or 3 the metering device can also be used to hold the test device 100 via contacts 18 which may be held securely in a strip port connector as understood by those skilled in the art.

In step 3, once removable portion 32' is free from upper surface 28, separable distal portion 16 can be rotated about hinge portion 30 in the direction of arrow D toward proximal portion 12. Removable portion 32', which is securely attached to separable distal portion 16 by seam 38 fixing non-removable portion 34' to upper surface 28, can be used as a handle to facilitate rotation of separable distal portion 16. Rotation of separable distal portion 16 about lower hinge portion 30 causes separable distal portion 16 (and associated removable portion 32 and non-removable portion 34A) to rotate (and if necessary detach from) the lower major surface of intermediate tip portion 14, exposing said lower major surface and the side walls of the periphery of intermediate tip portion 14.

In step 4, separable distal portion 16 and associated removable portion 32 and non-removable portion 34A of lower removable cover leaf 22 are preferably rotated, for example, up to almost 180° about hinge portions 30 so that separable distal portion 16, removable portions 32 and 32' and non-removable portion 34A provide an extended handle 40 for handling test device 100. Handle 40 can be used to assist insertion and/or removal of a test device 100 from a metering device. Following a test, the test strip 20, now contaminated with sample can be covered up by removable cover leaves 21 and 22, by rotating the separable distal portion 16 back to its initial position and the removable portions 32 and 32' about their hinge portions 30, 30' to re-cover intermediate tip portion 14.

Referring briefly to Figures 4 and 5, these show two additional embodiments of the invention broadly similar to those shown in Figures 1 and 2.

In Figure 4, test device 300 is seen from below having a first (upper) removable cover leaf 22 on upper major surface 28 having removable portion 32, a non-removable portion 34A fixedly attached to separable distal portion 16 of test strip 20 and a further non-removable portion 34B attached to the proximal portion 12. A second (lower) removable cover leaf 25 is provided on a lower major surface 29 of test strip 20. A distal portion 25A of cover leaf 25 may be omitted or may form a removable portion attached to and movable with removable distal portion 16 of test strip 20.

As in other embodiments, removable portion 32 may be hingedly and/or frangibly connected to non-removable portion 34B (e.g. by a line of weakness) and also provides a seal about intermediate tip portion 14 on the lower surface 29 of test strip 20 without being sealed to it. Thus, first removable cover leaf 22 has a removable portion 32 and a first non-removable portion 34A attached to separable distal portion 16 and a further non-removable portion 34B both selectively sealed (e.g. by welding or using adhesive) to upper major surface 28 of test strip 20 as described elsewhere herein. Upon movement (e.g. rotation) of separable distal portion 16 (e.g. by rotation of the U-shaped bendable frame forming the separable distal portion 16) in the direction of arrow B, removable portion 32 of the lower removable cover leaf 22 is also removed. Thus, the upper major surface 28' of intermediate tip portion 14, which bears test components in a test area, is unsealed ready for use (step 2), whilst the opposing surface of intermediate tip portion 14 remains covered by cover leaf 25. Separable distal portion16 may be rotated out of the plane of the remainder of the test strip 20 and used as a handle as described elsewhere herein.

Referring now to Figure 5, test device 400 is shown similar to that in Figure 2 having a first upper removable cover leaf 21' and a second lower removable cover leaf 22 as described in connection with Figures 1, 2 and 4 and elsewhere herein. The first upper removable cover leaf 21' has a removable portion 32" covering intermediate tip portion 14 and extending towards and partially over separable distal portion 16, and a non-removable portion 34" fixedly attached by robust and preferably permanent seam 38 to a proximal portion 12 of test strip 20. Thus the upper removable cover leaf 21', and lower removable cover leaf 22 along with the separable distal portion 16, are rotatable away from a distal end towards a proximal end of the test strip 20 in the direction of arrows G and B respectively. By rotating about respective hinge portions 30' and 30, for example up to almost 180°, until removable portions 32' and/or 32 (and separable distal portion 16 and associated non-removable portion 34A) lie alongside proximal portion 12, a handle 40' is provided by one or both of removable portions 32" and 32 (steps 1 to 4) (and separate distal portion 16 and non-removable portion 34A). Test device 400 may be inserted into a metering device prior to the removal of one and/or both removable portions 32', 32. The metering device may thus be used as a handle to hold the test device whilst removable portions 32', 32 are removed to expose intermediate tip portion 14. In such a circumstance rotation (e.g. by bending) through almost 180° may not be possible. As in other example embodiments, separable distal portion 16 is U-shaped surrounding the periphery of intermediate tip portion 14. Other shapes such as L-shaped (one side arm, one cross strut) or T-shaped (one central arm, one cross strut) or indeed other shapes may be envisaged in this and other embodiments.

In this and other embodiments, hinge portions 30', 30 may simply be bendable portions of the upper removable cover leaf 21' and/or lower removable cover leaf 22 and/or test strip 20 (between and/or part of proximal portion 12 and/or separable distal portion 16) or a hinge mechanism may be provided in one or more of these layers.

Embodiments of the invention can be envisaged in which separable distal portion 16 may be joined to proximal portion 12 of test strip 20 via a removable portion of a cover leaf, rather than being directly or indirectly connected via a test strip or being completely detachable. This and other arrangements in which separable distal portion 16 is not completely detachable facilitates subsequent replacement of the removable cover leaf and/or distal portion to cover up a used test area on intermediate tip portion 14.

Referring now to Figure 3, an alternative test device 200 is provided in which separable distal portion 16' is completely detachable along with one or both associated removable portions 132, 132' of upper and lower removable cover leaves 122, 124 respectively.

In Figure 3, the separable distal portion 16 is generally rectangular (although it may be other shapes e.g. square) and extends from one long side of the test strip 20 to the opposing side. The removable portion 132 of upper removable cover leaf 122 is rectangular, and along with an associated non-removable portion 134A is T-shaped broadly matching the size and shape of separable distal portion 16 and extending over and sealing a central portion of intermediate tip portion 14. Thus the removable portion 132 covers a test area comprising a sample inlet port 42, a sample chamber 44 and an air vent 46. In this example an 'end fill' test device 200 is shown but embodiments can be envisaged in which a 'side fill' test device is provided.

In this example embodiment, the sample chamber 44 (which may be a capillary chamber) may extend into and terminate in separable distal portion 16'. Separable distal portion 16' may be removed (in this case completely detached) by bending and breaking in the direction F about a longitudinal axis lying in the plane of the test strip from one short edge to another (in this an elongate test strip). In addition or alternatively the separable distal portion 16' may be detached by bending and breaking test strip 20 in direction E about a lateral axis from one long edge to another.

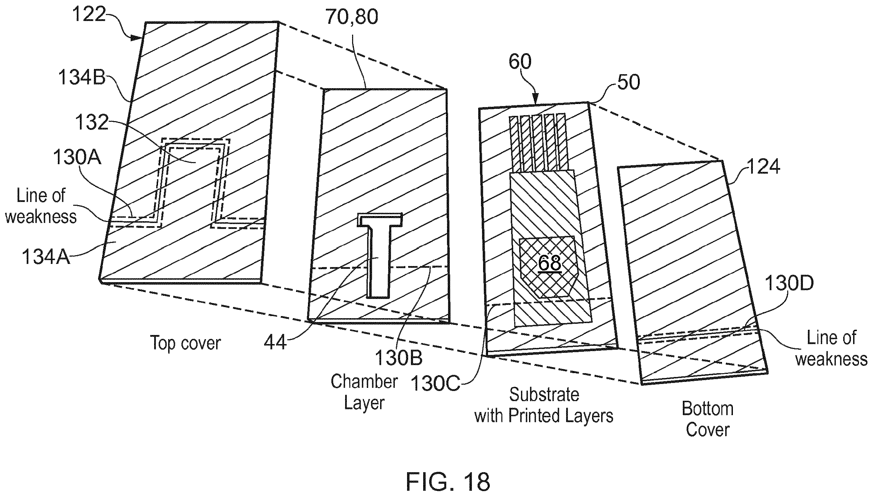

The chamber forming the sample chamber is split open, with that portion of it lying in the intermediate tip portion 14 forming the actual sample chamber 44 for receiving sample and an open end 42 of the sample chamber being formed in a side wall (here along a short edge) of test strip 20. This will be described in more detail later in connection with Figures 16 to 23.

Although not in accordance with the invention, referring now to Figure 24, test device 500 comprises a test strip 20 and an upper non-removable cover leaf 221 extending over an upper major surface 28 of test strip 20 and a lower non-removable cover leaf 222 extending over a lower major surface 29 of test strip 20. A test area (not shown) is formed on the upper major surface 28 on intermediate portion 14. Upper non-removable cover leaf 221 comprises a first non-removable portion 234A extending over and fixedly attached to separable distal portion 16 (in this example embodiment provided by a U-shaped frame surrounding intermediate portion 14). Typically first non-removable portion 234A is similarly U-shaped to match separable distal portion 16. Upper non-removable cover leaf 221 further comprises a second (preferably non-removable) portion 232 extending over and preferably fixedly attracted to intermediate portion 14. Upper non-removable cover leaf 221 may comprise a third non-removable portion 234B extending over and fixedly attached to proximal portion 12.

Typically upper non-removable cover leaf 221 is an integral component having first and second (234A, 232) or first, second and third non-removable portions (234A, 232 and 234B) being formed from a single piece of material. The same applies to lower non-removable cover leaf 222. Lower non-removable cover leaf 222 similarly comprises a first non-removable portion 334A extending over and fixedly attached to the lower surface of separable distal portion 16 of test strip 20, a second preferably non-removable portion 332 extending over and preferably fixedly attached to the intermediate portion 14, and also preferably a third non-removable portion 334B extending over and fixedly attached to proximal portion 12.

Upper first and second non-removable portions 234A, 232 may be frangibly connected to one another at lines of weakness therebetween (e.g. by utilising the kiss cutting method described elsewhere herein). Similarly the lower first and second non-removable portions 334A, 332 may be frangibly connected to one another at lines of weakness (e.g. by kiss cutting). Thus the upper and lower non-removable cover leaves 221, 222 are initially intact providing a seal over at least part or substantially all of the upper and lower major surfaces 28, 29 respectively and at least over the test area.

In one example embodiment, the upper second portion 232 may optionally not be attached to the intermediate portion 14 forming in effect a piercable removable portion 232, as will be described later. Intermediate portion 14 may be an intermediate tip portion as described elsewhere herein. Separable distal portion 16 may be a U-shaped frame as described elsewhere herein or may be rectangular or other shape attached to the intermediate portion 14 as described elsewhere herein. In step 1, a user has, preferably if appropriate, separated test device 500 from its neighbour(s) in a row. The user may insert the proximal end 12 into a meter (e.g. to engage exposed contacts 18).

The meter may comprise a protrusion (such as an elongate platform) (not shown) to engage the underneath of the proximal and intermediate portions 12, 14 of test strip 20 and associated lower third and second non-removable portions 334B, 332 of lower non-removable cover leaf 222

In step 2, gripping the meter (or proximal end 12, if the test device is not inserted in to the meter) in one hand, a user may depress the separable distal portion 16 of the test strip (typically by depressing the upper first non-removable cover portion 234A located thereon), causing the here U-shaped frame forming the separable distal portion 16 (and associated non-removable cover leaf portions 234A, 334A) to rotate downwards about hinge portion 30 in the direction of arrow B out of the plane of the remainder of test device 500.

During this step (step 2) the intermediate portion 14 remains in line with proximal portion 12. Also the upper first non-removable cover portion 234A breaks away from the upper second non-removable cover portion 232 on the intermediate portion 14. The second non-removable cover portion 232 remains on the intermediate portion 14.

Similarly, during step 2 the lower first non-removable cover portion 334A breaks away from the lower second non-removable portion 332 on intermediate tip portion 14. Thus the upper and lower major surfaces of the intermediate tip portion 14 remain covered (by non-removable portion 232 and 332 respectively), but the periphery of intermediate tip portion 14 is exposed. Thus, a sample inlet port (not shown) provided in the periphery of intermediate tip portion 14 (either as an 'end fill' or 'side fill' inlet port) is exposed. A cut away notch forming a depression and/or a protruding nodule forming a protrusion may be provided in the periphery of intermediate tip portion 14 to indicate the location of the sample inlet port to a user.

In the embodiment in which the intermediate tip portion is not attached to the upper cover leaf portion 232 of the upper cover leaf 221, during step 2, the separable distal portion 16 is rotated downwards in the direction of arrow B causing the upper cover leaf portion 232 to rotate with the remainder of upper cover leaf 221 in the direction of arrow B. Thus, the intermediate portion 14, here an intermediate tip portion 14, (which does not rotate and is preferably supported on a platform on the meter) exerts an upward force on upper cover leaf portion 232 and pierces it causing it to break. The (now broken) upper cover leaf portion 232 travels downwards along with intermediate tip portion 14 in the direction of arrow B. Thus, intermediate tip portion 14 (its upper surface and its periphery) is exposed ready to receive a sample.

Typically, in either case, if a platform is provided on a meter adjacent to the strip port connector, the platform will approximately match the size and shape of the intermediate portion 14, when the strip is inserted into the strip port connector of the meter thus providing support to the intermediate portion 14 during the rotation of separable distal portion 16.

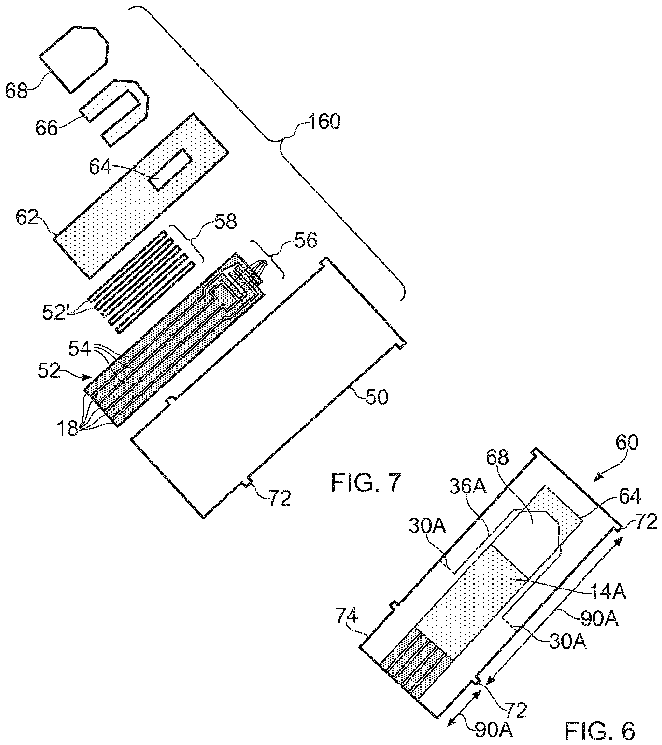

Referring now to the invention, Figures 6 to 23 show example embodiments to illustrate the invention and describe, by way of example only, a single use blood glucose test strip that electrochemically determines analyte presence or concentration in blood.

Referring to Figures 6 and 7, each test strip 20 comprises a base substrate 50 on an upper major surface of which is disposed a conductive layer 52 comprising carbon tracks 54 forming carbon pads 56 and electrical contacts 18. Preferably, a second conductive layer 52' is provided comprising silver tracks 58 overlying part of carbon tracks 54. Next, an insulating layer 62 is overlaid the conductive layer(s) 52, 52', leaving an insulation window 64 to define the width of the carbon pads 56 within the test area. Next a shaped adhesive layer 66; and an appropriately shaped reagent pad 68 carrying reagent mixture are located over insulation layer 62 and insulation window 64. Thus measurement electrodes (not shown) are formed by carbon pads 56 overlaid with reagent pad 68 via insulation window 64. Thus a base substrate with printed layers 60 is formed (see Figure 6).

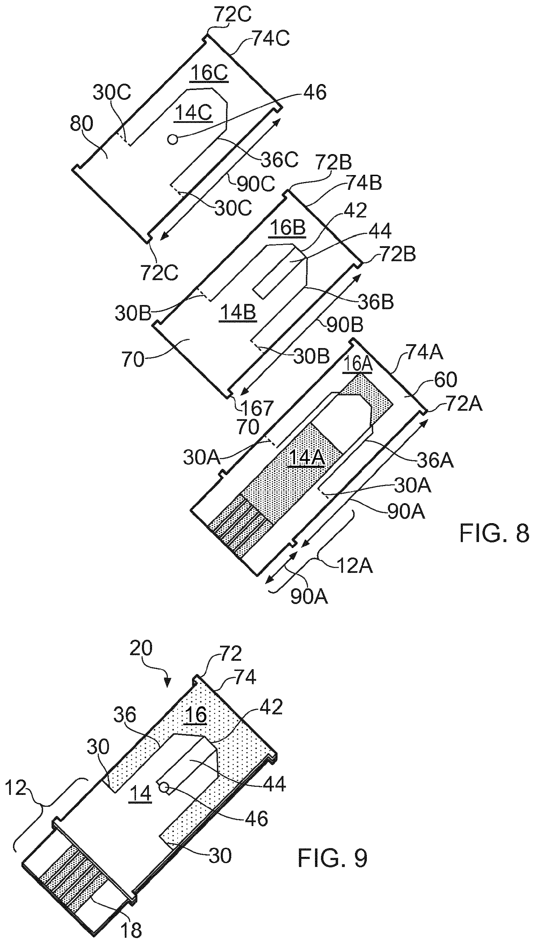

Referring now to Figures 8 and 9, the now formed base substrate with printed layers 60 and a spacer layer 70 and a hydrophilic layer 80 are laminated together to provide test strip 20. Hydrophilic layer 80 may also be known as an anti-fog layer. Exposed contacts 18 are located on a proximal portion 12 of test strip 20. An intermediate portion 14 comprising test strip components (for example measurement electrodes, sample inlet port 42, sample chamber 44 and air vent 46) in a test area is provided. Typically, the measurement electrodes (here carbon pads 56 overlaid with reagent (such as a printed reagent layer or reagent pad 68)) are located on a lower floor of the sample chamber 44 with the sample inlet port 42 being at a distal end of the sample chamber and the air vent 46 being at a proximal end of the sample chamber.

In the example embodiment, the test strip 20 has junctions 72 (typically frangible portions of substrate 50 and/or removable cover leaf/leaves - see items 21 and 22 in Figures 12 and 13) on its perimeter 74 connecting test strip 20 to its neighbours. Test strip 20 has a U-shaped bendable frame (forming separable distal portion 16) provided within the perimeter 74, the frame substantially surrounds an intermediate portion 14. Separable distal portion 16 is capable of being deflected (bent away from) intermediate portion 14. Here an intermediate tip portion 14 is provided having a narrower width than the proximal portion 12. Here, the separable distal portion 16 has two laterally spaced arms initially lying alongside the narrower intermediate tip portion 14 and a cross-strut forming a frame surrounding intermediate tip portion 14. The size and/or shape of the outer periphery of the intermediate tip portion 14 corresponds to the size and/or shape of the inner periphery of the separable distal portion 16 (here U-shaped bendable frame 16) preferably so that these abut one another. Indeed, preferably the intermediate tip portion 14 and separable distal portion 16 (and also preferably the proximal portion 12) of the test strip 20 are formed from the same integral substrate 50 (for example having cuts 36) and/or hinge portions 30 formed in the substrate 50 as appropriate to define the different portions). The intermediate tip portion 14 has on or within its perimeter an opening forming sample inlet port 42 in fluid communication with a sample chamber 44. Sample chamber 44 is in fluid communication with an air vent 46. Contacts 18 are capable of communicating with an external metering device.

Referring now to Figures 10, 12 and 13 a profiled upper seal 48/48' is shown bonding an upper (for example a first) removable cover leaf 21 to an upper major surface 28 of test strip 20. A rectangular shaped portion of the profiled seal (here a distal seam 38) permanently joins the upper removable cover leaf 21 to a corresponding portion of the upper major surface 28 of separable distal portion 16 of test strip 20. The upper removable cover leaf 21 is sealed at least in part to at least part of the remainder of the bendable frame (removal distal portion 16), and indeed in this example embodiment to at least part of part of the proximal portion 12, in such a way that it can be removed by a user for example by peeling off. This is termed the removable portion (32' in Figure 2) of upper removable cover leaf. The seal may be an adhesive seal (formed from an adhesive layer) or a weld (e.g. thermoplastic weld) or other join as known to those to skilled in the art.

The upper profiled seal 48/48'is shaped preferably to completely surround the intermediate tip portion 14 without sealing to intermediate tip portion 14 to any appreciable extent. Nevertheless, the upper profiled seal may seal upper removable cover leaf 21 partially within the perimeter of the intermediate tip portion 14 (e.g. across cuts 36) as described elsewhere herein. Upper removable cover leaf 21 may for example be removably sealed to upper surface 28 in the region of its removable portion by provision of upper seal 48/48' as a peelable seal, peelable adhesive or peelable weld (e.g. a thermal weld). Proximal portion 48' of the upper profiled seal may have a pointed shape to facilitate the start of a peeling action. An unsealed tab portion 26 on upper removable cover leaf 21 is provided to aid removal of the removable portion of the upper removable cover leaf 21.

Referring now to Figures 11, 12 and 13, a lower profiled seal 49 bonds a lower (for example a second) removable cover leaf 22 to the test strip 20 in manner so that the seal 49 does not seal the removable cover leaf 22 (in the region of removable portion 32 - see Figure 2) to the intermediate tip portion 14 or not to any appreciable extent. The lower profiled seal 49 may be shaped to achieve this or may have lower quality sealing properties in the region of intermediate tip portion 14. The seal 49 preferably seals the lower removable cover leaf 22 to the rest of the lower surface of the test strip 20 and in particular to the separable distal portion 16 and preferably the proximal portion 12) so as to surround the intermediate tip portion 14. This may be a permanent seal for example a permanent weld or other permanent joint.

Having had upper and lower removable cover leaves 21 and 22 mounted thereon via respective seals 48 and 49 (which may be provided by separate adhesive layers or regions of thermal or chemical sealing of removable cover leaves 21, 22), a test device 100 comprising a now sealed test strip 20 is provided as shown in Figure 13. The upper removable cover leaf 21 is a moisture barrier and/or moisture adsorbing laminate, and/or the lower removable cover leaf 22 is a moisture barrier and/or moisture adsorbing laminate. One, or preferably both, removable cover leaves comprise a desiccant e.g. in a layer of desiccant loaded plastic.

Whilst the upper and lower removable cover leaves 21 and 22 cover, surround and protect the intermediate tip portion 14 from exposure to moisture in the atmosphere via major surfaces 28 and 29 of test strip 20, the edges of the test strip 20 about its perimeter 74 are not covered. Thus moisture may be absorbed into the test strip via this exposed perimeter. The inventors have appreciated that this is a limited route for introduction of moisture into the strip and such moisture introduction may be offset by use of removable cover leaves having moisture adsorbing properties and/or a supplementary carrying pouch which may have moisture adsorbing properties and/or other measures. Indeed, sealing across and partially within cuts 36 via one or both major surfaces may further impede moisture ingress by providing partially sealing of the side wall forming the periphery of intermediate tip portion 14 (having the test components).

The following describes how to use the test device of the invention. Rows of a limited number of connected test devices 10 (e.g. as in Figure 23) may be manufactured and subsequently stored in a flow wrapped moisture barrier proof pouch (not shown). Each row may contain multiple test devices joined together to form a user test card by a series of junctions 72 around the perimeter 74 of each test device 10. Typically 2, 3, 4, 5 or 6 test devices may be provided in a user test card preferably in the form a row. The pouch is designed to be easily opened so that a user test card, of e.g. a single row of test devices 10, can be removed.

A person may remove one or more test devices 10 from the row by holding the test device 10 to be removed in one hand and the remainder of the row of test devices in the other hand. A twisting or a pulling motion between the two hands breaks the junctions 72 between the devices 10 held in each hand so that the test devices 10 can be separated.

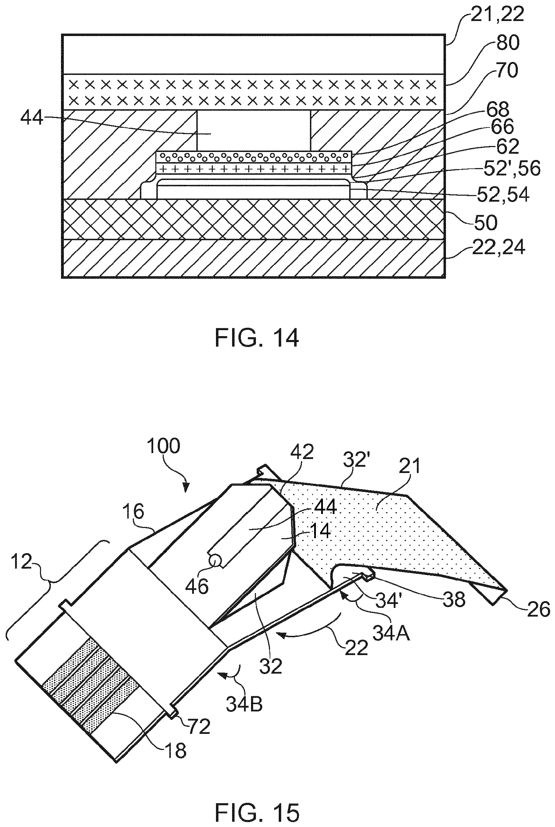

Referring now to Figure 15, a single use test device 100 can be held at the proximal end 12 near contacts 18 by the fingers of one hand while gripping the tab 26 of top cover 21 by the fingers of the other hand. The tab 26 is pulled in the direction of the tip in a peeling motion. The top cover 21 peels back as far as the portion of the bendable frame (separable distal portion 16) adjacent sample port 42. The top cover 21 does not peel back any further because non-removable portion 34' is attached robustly to that portion of the bendable frame by a seam 38.

Next, while still holding the tab portion 26 of top cover 21 and/or the removable portion 32' of top cover 21, top cover 21 can be used to provide a handle and additional leverage to bend the bendable frame (separable distal portion 16) back and away from the tip (intermediate tip portion 14).

The test device 100 in figure 15 is shown part way through this process. Holding the peeled-back top cover 21 and/or gripping the now bent back bendable frame (separable distal portion 16) and/or gripping the sample application tip 14, the proximal portion 12 of test strip 20 can be inserted into an external metering device (not shown).

The present invention advantageously enables the sample inlet port 42 to be made accessible by the bending of the bendable frame 16 away from the tip. Thus, a fluid sample can now be easily presented to the sample inlet port 42 in the end wall of the intermediate tip portion 14. The fluid sample is drawn into the sample chamber 44 by capillary action with chamber gases being vented through the air vent 46.

An assay is conducted on the fluid sample. When the assay is complete, the test device 100 can be removed from the external metering device by gripping the tab portion 26, and/or removable portion 32 of the top cover 21 and/or bendable frame (separable distal portion 16) and pulling the test device 100 out of the metering device.

There is an option to bend the bendable frame (separable distal portion 16) back into its initial position in which it surrounds the intermediate tip portion 14 so that top cover 21 can be laid across the upper major surface of the device so as to provide protection against touching any residual fluid sample adhered to exposed surfaces or the edges of the intermediate tip portion 14. The test device 100 is ready for safe disposal.

The following describes how to manufacture a test device. Referring to Figures 6 to 13, a printed substrate with layers 60 is formed from a first sheet-like insulating substrate material in a web of suitable width and length (forming base substrate 50).

A first series of carbon tracks 54 forming a first conductive layer 52 is disposed on a first major surface of base substrate 50. At one end conductive tracks 54 form carbon pads 56 which form part of the measurement electrodes. A second series of conductive tracks (silver tracks 58) form a second conductive layer 52' disposed on and substantially covering part of carbon tracks 54. A first portion of the carbon tracks 54 is not covered by the silver tracks 58 forming the carbon pads 56 in a working area of the test strip 60. A second portion of the carbon tracks 54 is not covered by the silver tracks 58 forming the electrical contacts 18 that communicate with an external metering device.

An electrically insulating layer 62, formed of e.g. dielectric material, is disposed on first (here upper) major surface of the base substrate 50 and substantially covers the carbon/silver tracks 54, 58. A first portion of the carbon tracks 54 defined by insulation window 64 is not covered with insulating layer 62 completing the definition of the test strip working area. The contacts 18 are not covered with insulating layer 62. An adhesive layer 66 is disposed on and partially covers the insulating layer 62 and substantially surrounds the working area of the test strip 60. Adhesive layer 66 is U-shaped to avoid blocking access to the working area within insulation window 64. A reagent layer 68 disposed on the adhesive layer 66 and substantially covers the working area within insulation window 64. The completed substrate with printed layers 60 is seen in Figure 6. A first series of cuts 90A are made through the thickness of the printed base substrate 60 defining the perimeter 74 of and a series of junctions 72 of base substrate 50 are not cut so that each printed base substrate 60 remains connected to its neighbour. These cuts 90A may be made before or after printing on base substrate 50 to form printed base substrate 60 with printed layers. A second series of cuts 36A through the thickness of the base substrate within the perimeter 74 partially surrounds the carbon pads 56 in the working area to define the base substrate portion 14A of a sample application tip (intermediate tip portion 14) and the base substrate portion 16A of a bendable frame (separable distal portion 16) substantially surrounding the intermediate tip portion 14A.

First and second series of cuts 90A, 36A may be formed simultaneously or one preceding another. Lines of weakness 30A may be provided in base substrate 50 to provide hinge portions between the laterally spaced side arms of the bendable frame (separable distal portion 16A) and the proximal portion 12A bearing contacts 18.

Next, a second sheet-like insulating substrate material of about 50mm wide by about 1 kilometre in length by 0.100mm thick is provided to from a spacer layer 70 (see Figure 8). Typically the spacer layer 70 has adhesive disposed on its first and second major surfaces (not shown). Spacer layer 70 may be pre-cut to have first series of cuts 90B through the thickness of the spacer layer 70 separating the outline of the spacer layer precursor for one test strip from its neighbour (forming perimeter 74B) and defining a series of inter-connecting junctions 72B so that each spacer layer precursor remains connected to its neighbour.

A second series of cuts 36B through the spacer layer within perimeter 74B defines the spacer portion of a sample application tip (intermediate portion 14B) and the spacer portion of a bendable frame (removal distal portion 16B) substantially surrounding the intermediate tip portion 14B.

A third series of cuts through the spacer layer within the perimeter 74B of the intermediate tip portion 14B creates a hole or void defining the spacer portion of a sample chamber 44. Sample chamber 44 may be pre-formed in the spacer layer rather than being cut on the fly.

Next, a third sheet-like insulating substrate material of about 50mm wide by 1 kilometre in length by 0.100mm thick is provided to form a hydrophilic layer 80 (also known as an anti-fog layer). The hydrophilic layer 80 comprises a hydrophilic coating (not shown) disposed on a major surface of hydrophilic layer 80 facing the test area defined by sample chamber 44 and insulation window 64. The hydrophilic layer 80 may also comprise an adhesive or heat activated binding polymer coating disposed on a second major surface (facing outwards). A first series of cuts 90C defines the perimeter 74C with a series of junctions 72 remaining so that each test strip portion of spacer layer remains connected to its neighbour.

A second series of cuts 36C may be made within the perimeter 74C to define the anti-fog portion of a sample application tip (intermediate tip portion 14C) and the anti-fog portion of a bendable frame (separable distal portion 16C) surrounding the intermediate tip portion 14C.

A third series of cuts may be made through the antifog layer to create a hole or void within the perimeter of the tip defining the sample chamber air vent 46. Air vent 46 may be pre-formed in hydrophilic layer 80. Indeed, first, second and third series of cuts may be pre-formed in base substrate 50, spacer layer 70 and/or hydrophilic layer 80 rather than being cut on the fly.

The outer facing major surface of the spacer layer 70 (for example with the adhesive) is bonded to the inner facing major surface of the hydrophilic layer 80 in register such that: the rectangular cut-out or void of the spacer layer defines the width of the sample chamber 44; the air vent 46 on the hydrophilic layer 80 lies at one end of the sample chamber 44 (typically the proximal end); the outer perimeters 74B, 74C of the spacer layer 70 and hydrophilic layer 80 match when these are laminated; the junctions 72B, 72C on the spacer layer 70 and hydrophilic layer 80 are of the same geometry so that these match when these are laminated; and the spacer layer and hydrophilic layer tip portions 14B and 14C are similarly sized and shaped so that these match when these are laminated.

Next, the upper major surface 28 of the printed base substrate 60 is bonded to and is substantially covered by the spacer layer/hydrophilic layer laminate to form a test strip 20 such that: typically, these are of substantially the same size and are in register so that: the contacts 18 are not covered; the sample chamber 44 is fully defined; the perimeter 74A on the base substrate with printed layers 60, that defines the test strip width, matches the perimeter 74B, 74C of the spacer/hydrophilic laminate; the geometry of the intermediate tip portion 14A on the printed base substrate 60 matches the geometry of the intermediate tip portion 14B, 14C on the laminate and the bendable frame 16 that surrounds the intermediate tip portion 14 is fully defined from portions 16A, 16B and 16C.

Referring now to Figures 10 to 15 and especially to Figures 10 to 13, a fourth sheet-like insulating substrate material of about 50mm wide by 1 kilometre in length by 0.150mm thick is provided to form top cover 21 (upper removable cover leaf 21). Typically, this is a multi-laminate material with moisture barrier properties and/or moisture adsorption properties. Optionally, upper removable cover leaf 21 is a laminate such as a triple layered laminate and may comprise a durable plastic as an outside facing layer, a thermo-formable material that may have a desiccant material as part of its structure as an inside facing layer (facing inward towards test strip 20) and a high moisture barrier film between the outside and the inside facing layers. Preferably, the inside facing surface of the upper removable cover leaf 21 is laminated to a first major surface 28 of the test strip 20 so that the inside facing layer of the upper removable cover leaf 21 comes into contact with major surface 28 of the test strip. Furthermore, preferably the upper removable cover leaf 21 optionally completely covers the first major surface 28 of the test strip and extends to substantially or completely cover the exposed carbon electrode contacts. Optionally, a tab 26 may remain unsealed to but nominally extends over proximal portion 12 in the region of contacts 18.

As an alternative to a separate adhesive layer to hold removable cover leaf 21 in place, a profiled heat seal may be used. Thus a next step may include applying a profiled heat seal so that: an area of the upper removable cover leaf 21 is selectively sealed to an area 48 of the test strip 20 such that the bendable frame (separable distal portion 16) is substantially sealed to the top cover leaf 21; the intermediate tip portion 14 is partially sealed to the top cover and is completely surrounded by a seal; and the profile of the seal is designed to facilitate peeling of the upper removable cover leaf 21 off the test strip 20 (for example, a reduced seal or non-existent seal in the region of removable portion 32'). Preferably, the profile and/or heat seal process parameters are such that the upper removable cover leaf 21 remains attached by a portion 34' to the bendable frame (separable distal portion 16) of the test strip 20.

A fifth sheet-like insulating substrate material of about 50mm wide by 1 kilometre in length by 0.150mm thick is provided to form bottom cover 22 (lower removable cover leaf 22). Typically, this is a multi-laminate material with moisture barrier properties and/or moisture adsorption properties and structure as described above in connection with the upper removable cover leaf 21. Thus, an inside facing surface of the lower removable cover leaf 22 is laminated to a second major surface 29 of the test strip 20 so that the inside facing layer comes into contact with lower major surface 29 of the test strip 20, preferably completely covering it.

A next step (if a separate adhesive layer is not used) may be applying a profiled heat seal 49 so that an area 49 of the bottom cover is selectively sealed to an area of the test strip 20 so that: the bendable frame (separable distal portion 16) is substantially sealed to the lower removable cover leaf 22; the intermediate tip portion 14 is not sealed (or not sealed to any appreciable extent) to the removable cover leaf 22 (in the region of removable portion 32), and the intermediate tip portion is completely surrounded by a seal.

A series of cuts 90D through the upper/lower removable cover leaves 21 and 22 define their perimeters 74'/74" with substantially the same geometry as the perimeter 74 (74A, 74B,74C) defined by cuts on test strip 20. Optionally one or more cuts 90A, 90B, 90C and 90D may be formed at the same time, for example, after formation of a packaged test device 10, 100, 200, 300, 400.

Finally, a card of packaged test devices 10, 100, 200, 300, 400 (see for Example Figures 22 and 23) may be cut into a user test card comprising a row of test devices (e.g.2, 3, 4, 5 or 6) and optionally packaged in a secondary packaging such as flow wrapped high moisture barrier laminated material, such as a desiccant loaded pouch (preferably re-sealable). The pouch may provide an additional moisture barrier to keep such rows of test devices 10, 100, 200, 300, 400 stable while these are in the pouch. The upper and lower removal cover leaves 21 and 22 provide environmental protection for each individual test device 10, 100, 200, 300, 400 after removal from the pouch for a prolonged period of time so that individual test devices can be taken and stored in a non-protective container e.g. a pocket or a wallet for journeys or extended periods away from home. The junctions 72/72' between test devices 10, 100, 200, 300, 400 provide a means to keep materials and test devices in web format. This provides for a packaging method that packages individual test devices 10 while they are still attached to each other in web format or as an array (e.g. a user test card) of test devices 10, 100, 200, 300, 400 removed from the web. This reduces the cost of manufacture.

The bendable frame (separable distal portion 16) provides a sealing platform for the upper removable cover leaf 21, 21', 22,. In particular, the bendable frame (separable distal portion 16) bends out of the plane of the test strip 20 and enables the manufacture of a test device 10 with an end fill sample chamber. In another embodiment there may be (not illustrated) a sample inlet port or an upper major surface of the test strip which provides for a top fill type sample chamber. The tab 26 (of the upper removable cover leaf 21) provides for an easy means to peel the top cover away from the intermediate tip portion 14. Furthermore, the upper removable cover leaf 21 is strongly sealed or welded to the portion of the bendable frame (separable distal portion 16) adjacent the sample inlet port 42 which means that the upper removable cover leaf 21 remains an integral part of the test device so that the tab 26 and peeled back top cover (removable portion 32') can be used as a handle to hold the strip and/or a handle to pull the strip out of an electronic device and/or a handle to assist in bending back the bendable frame (separable distal portion 16) and/or a way of covering the used test strip for clean disposal and therefore this improves the ease of use of access to individual test strips.

Referring to Figures 16 to 21, these show an alternative test device 200 according to a further aspect of the invention having an integrated (optionally twist off) removable cap forming a separable distal portion 16. Test device 200 is rectangular, having two long edges and two short edges, and substantially planar having upper and lower major surfaces. Test device 200 has an intermediate portion 14' and a proximal portion 12 (here bearing exposed contacts 18). Furthermore test device 200 comprises upper and lower removable cover leaves 122, 124 respectively.

Initially, proximal portion 12, intermediate portion 14' and separable distal portion 16' form an integral component, here being formed from the same test strip 20. However, as in other embodiments of the invention, two or more of the three portions (for example two or more of the three portions of the test strip 20 or of the underlying base substrate 60) may be formed separately and joined together in an appropriate manner to provide integrated test device 200. Here, preferably the periphery of intermediate portion 14' forming a long edge of test strip 20 is in line with the periphery of proximal portion 12 forming a long edge of test strip 20. Thus, in contrast with the embodiment shown in Figure 13, intermediate portion 14' is of the same or substantially the same width (long edge to long edge) as that of proximal portion 12. Also, separable distal portion 16' is preferably the same or substantially the same width as intermediate portion 14'.

Cuts 130 are formed part way across the width of test device 200 from each long edge towards a test area 27 comprising test components as described elsewhere herein but stopping short of the test area 27. These cuts 130 may be pre-formed in test strip 20 and/or upper and/or lower removable cover leaves 122 and 124 or these may be formed in these once test device 200 is assembled, (although preferably these are formed prior to singulation into individual test devices or separation into a small row of test devices).

Upper removable cover leaf 122 (described below as top cover 122) comprises a removable portion 132 peelably covering the test area 27, a non removable portion 134A fixedly attached to separable distal portion 16' and a further non-removable portion 134B fixedly attached to the intermediate portion and the proximal portion and. Removable portion 132 of upper removable cover leaf 122 extends over and surrounds the test components, sealing these in respect of the upper surface of the test device 200 from moisture ingress. Upper removable cover leaf 122 also preferably comprises a non removable portion 134B covering much of the remainder of test strip 20. Optionally, non removable portion 134B can be dispensed with and the removable portion 132 of upper removable cover leaf 122 may extend to cover and seal only test components or may extend beyond to seal at least part of or substantially all of the upper surface of test strip 20, although contacts 18 may be left exposed.

One embodiment of the test device 200 construction is as follows. A printed substrate layer 60 with printed electrochemical layers is formed, including a reagent pad 68 on a base substrate 50. A combined spacer/hydrophilic layer 70/80 defines a sample chamber 44 and preferably substantially covers the upper major surface of the printed substrate layer 60 of test strip 20, although preferably it does not cover contacts 18. A bottom cover 124 (lower cover leaf 124) with moisture vapour barrier properties preferably completely covers the underside of the substrate layer of test strip 20. A top cover 122 with moisture vapour barrier properties, preferably completely covers the upper major surface of spacer layer 70/80.

Also in a preferred embodiment of this aspect of the invention, a further important feature of the test device 200 is that the top cover 122 is divided into two sections (a first section comprising removable portion 132 and non-removable portion 134A and a second section comprising non-removable portion 134B (either pre-formed as separate two sections or cut at 130A to form two sections in an integral cover leaf, or provided with lines of weakness at 130A). This allows for the section (removable portion 132 and non-removable portion 134A) of the top cover 122 that covers (here directly covers) the sample chamber 44 to be removed (along with separable distal portion 16) to expose the chamber visually to the user and for the air vent 46 at the end of the sample chamber to be exposed physically to allow sample chamber gases to be vented when fluid sample is being introduced into to the chamber. The sample chamber opening (sample inlet port 42) may be provided in the hydrophilic layer, (similar to air vent 46) to provide a 'top fill' sample inlet port, in which case this would be exposed upon removal of removal cover portion 132. Alternatively the sample inlet port 42 may be provided in a side wall along a long edge ("side fill") or preferably a short edge ("end fill") of test strip 20 as shown in the Figures.

The sample chamber opening (sample inlet port 42) is covered (albeit it is not in direct contact with top cover 122) by top cover 122 and is also exposed physically, when the twist off cap (separable distal portion 16') is broken off and removed along with removable portion 132.

In a preferred embodiment, the top cover 122 can be constructed or processed such that the robust outer material of the multi-laminate thin film forming the top cover 122 is cut but not the middle moisture barrier (e.g. aluminium layer) and/or not the inner facing tie (e.g. thermoplastic layer) althoughthe inner facing thermoplastic layer may also be cut. Thus top cover 122 is provided with lines of weakness (here cuts 130A) to facilitate it breaking into two sections (a first section comprising removable portion 132 and non-removable portion 134A and a second section comprising non-removable portion 134B). The profile of cuts 130A can be matched with the portion of test device required to be exposed when the twist off cap is separated from the test device. The moisture barrier properties of the top layer 122 are not affected because the moisture barrier layer of, for example, aluminium is not cut. This method of cutting material is known as kiss cutting.

The lower cover leaf 124 (described below as bottom cover 124) can be, for example, a multi-laminate thin film material whereby a moisture barrier layer such as aluminium is sandwiched between a robust outer layer of plastic exposed to the outside environment and an internal tie layer such as adhesive or thermoplastic that binds to the substrate layer 50. The top cover 122 can be the same material as the bottom cover. Top cover 122 may completely cover the chamber layer.

The substrate layer 50 can be formed, for example, from a continuous web of a heat stable polyester that can be continuously printed reel to reel to lay down and dry several important electrochemical layers such as conductive layer(s) 52, 52' including contacts 18, an insulation layer 62, and a reagent layer 68 in the form of a pad containing the reagent mixture that is placed to form a reaction zone within insulation window 64 on the intermediate portion 14' of the test strip 20.

A spacer layer 70 defines a channel with capillary action properties. This channel is placed directly over the reaction zone of the intermediate portion 14' creating a sample chamber 44. An air vent 46 (here a rectangular air vent to aid registration with the chamber 44 during manufacturing) is cut through the thickness of a hydrophilic layer 80 at one end of this channel allowing sample chamber gases to be vented when sampling fluid to do a test. Preferably in one important embodiment, the channel extends from the intermediate portion 14' into the twist off cap component forming separable distal portion 16'.

Preferably the separate layers 124, 60, 70, 80, 122 are brought together in a continuous reel to reel process from reel stock to produce a single web construction. An example section of a completed web of test devices 200 is shown in Figure 22 with cuts 90 in between neighbouring devices. Frangible interconnecting junctions 72 may be formed in one or more of the various layers (not shown).

A cap forming step can be introduced whereby material is cut away at 130 between the twist off cap portion (separable distal portion 16') and the sample testing portion (here formed by proximal portion 12 and intermediate portion 14' of the test device 200). This feature provides a good visual cue to the user to distinguish between the portions of the test strip. The cuts outs formed by cuts 130 can also be designed in such a way as to facilitate twisting off the cap portion (separable distal portion 16') and separating it from the remainder of the test device by creating a weak point in the test device 200. The narrow neck formed by cuts 130 between intermediate portion 14' and separable distal portion 16'forms a region of weakness in test device 200. Figure 22 shows a sample array of fully constructed test strips taken from a reel alongside a singulated test strip taken from that array. The cut outs 130 created during the cap forming process are highlighted.

In a preferred embodiment a feature of the invention is that the channel defining the sample chamber 44 extends from the sample testing portion (specifically form the intermediate portion 14') of the test device 200 into the twist off cap portion (separable distal portion 16'), see Figures 19, 20 and 21). The channel is designed in such a way that it defines the sample chamber 44 and extends into the twist off cap portion (separable distal portion 16') of the test device 200.

When the twist off cap (separable distal portion 16') is separated from the remainder of the test device 200 it results in the creation of an opening 42 in the channel to the outside environment which allows entry of the fluid sample to be analysed, thus forming the sample inlet port 42.

A preferred embodiment of one aspect of the invention is described in which the end user receives a block of individually packaged test strips attached together as shown in Figure 23. The block can be any number of test strips but it is preferred to supply blocks of between 5 and 100 strips. A test strip can be singulated from the block by twisting or breaking the strip away from the block in one easy step. This can be facilitated by including perforations or cuts between test strips.

Test strips 20 are packaged individually to protect them from moisture vapour while providing a simple, easy to open, solution to access the test strip to do a test.

In various aspects, the invention provides a means to open the test strip to create an opening to a sample chamber, a means to visually expose the sample chamber and to physically expose an air vent in one easy step. Furthermore, aspects of the invention provide a way of integrating a cap forming process into a reel to reel lamination process to form a break and/or twist off cap. Furthermore aspects of the invention provide individually packaging of the test strips in such a way that may leave exposed the printed electrical contacts of the test strip contacts with a meter. In various example embodiments, the invention provides packaging configurations that improve protection of each strip individually from the effects of moisture and/or reduces the cost and complexity of manufacture and/or improves the ease of use of accessing individual test strips. The present invention provides individually packaged test strips that are easy to open while providing an efficient and cost effective method of mass production.

## Claims

1. A test device (10, 100, 300, 400) comprising:
a test strip (20) having first and second major surfaces (28, 29) and comprising:
- a proximal portion (12) for bearing contacts (18) for a metering device,
- an intermediate portion (14) for receiving a sample comprising a test area on the first major surface (28), the test area comprising at least one test component;
- a separable distal portion (16); separable so as to be movable with respect to at least the intermediate portion (14);
and in which the test device (10, 100, 300, 400) further comprises:
at least one removable cover leaf (21, 22) comprising a moisture barrier layer;
the at least one removable cover leaf (21, 22) comprising a removable portion (32, 32') extending over at least part of one of the major surfaces (28, 29) of the test strip (20) so as to seal the test area from moisture ingress and,
the at least one removable cover leaf (21, 22) further comprising a non-removable portion (34A, 34') attached to the separable distal portion (16) of the test strip (20).

2. A test device (10, 100, 300, 400) according to claim 1 in which the removable portion (32, 32') of the removable cover leaf (21, 22) extends over the first major surface (28) of the test strip (20) to seal the test area in respect of the first major surface (28), or in which the removable portion (32, 32') of the removable cover leaf (21, 22) extends over the second major surface (29) of the test strip so as to seal the test area in respect of the second major surface (29).

3. A test device (10, 100, 300, 400) according to claim 1 or 2 in which the separable distal portion (16) of the test strip (20) is rotatable with respect to the intermediate portion (14) and/or to the proximal portion (12) of the test strip (20),
and/or,
in which the separable distal portion (16) of the test strip (20) is connected to the intermediate portion (14) and/or proximal portion (12) of the test strip (20) by one or more hinge portions (30, 30').

4. A test device (10, 100, 300, 400) according to any preceding claim in which the separable distal portion (16) comprises at least one arm lying alongside the intermediate portion (14) of the test strip (20),
and/or,
in which the separable distal portion (16) forms a frame (16) about the intermediate portion (14) of the test strip (20).

5. A test device (10, 100, 300, 400) according to claim 4 in which the separable distal portion (16) is U-shaped having two laterally spaced arms lying alongside the intermediate portion (14) and a distal cross strut therebetween to form the frame (16) about the intermediate portion (14),
and/or,
in which a hinge portion (30, 30') is provided between one or each arm and the proximal portion (12), or between the frame (16) and the proximal portion (12).

6. A test device (10, 100, 300, 400) according to any preceding claim in which the size and/or shape of the periphery of the separable distal portion (16) opposite the intermediate portion (14) corresponds with the size and/or shape of the opposing periphery of the intermediate portion (14) whereby these lie immediately adjacent one another.

7. A test device (10, 100, 300, 400) according to any preceding claim in which the intermediate portion (14) is of narrower width than the proximal portion (12) and forms an intermediate tip portion (14) comprising a tip,
and/or,
in which the separable distal portion (16) of the test strip forms a frame (16) about the intermediate portion (14) of the test strip (20), and the removable portion (32, 32') of the removable cover leaf (21, 22) extends over at least the intermediate portion (14) and the non-removable portion (34A, 34') of the removable cover leaf (21, 22) extends over the frame (16).

8. A test device (10, 100, 300, 400) according to any preceding claim in which the removable portion (32, 32') of the removable cover leaf (21, 22) extends i) over at least part or all of the intermediate portion (14), or ii) over at least part or all of the intermediate portion (14) and over at least part or all of the proximal portion (12).

9. A test device (10, 100, 300, 400) according to any preceding claim, in which:-
i) the removable portion (32, 32') of the at least one removable cover leaf (21, 22) comprises pre-selected regions attached to the test strip (20) surrounding at least part or all of the intermediate portion (14), or surrounding at least part or all of the test area, of the test strip (20), and,
the pre-selected regions of the removable portion (32, 32') attached to the test strip (20) are attached in a manner so that at least part or all of the removable portion (32, 32') of the removable cover leaf (21, 22) is not attached or is not attached to any appreciable extent to the intermediate portion (14), or is not attached or is not attached to any appreciable extent to the test area, of the test strip (20),
or,
ii) in which the non-removable portion (34A, 34') of the at least one removable cover leaf (21, 22) is permanently attached to the separable distal portion (16) of the test strip (20) and the removable portion (32, 32') is peelably attached to at least the intermediate portion (14) of the test strip (20).

10. A test device (10, 100, 300, 400) according to any preceding claim in which the test strip (20) comprises a substrate (50) and the substrate (50) is an integral component and comprises a proximal portion (12A), an intermediate portion (14A) and a separable distal portion (16A),
and/or,
in which the substrate (50) comprises one or more lines of weakness (30A) and/or cuts (36, 36A) delineating between the intermediate portion (14) and the separable distal portion (16), and/or,
in which the substrate (50) comprises one or more lines of weakness (30A) delineating between the proximal portion (12) and the separable distal portion (16),
and/or,
in which the at least one removable cover leaf (21, 22) comprises one or more lines of weakness (30A) and/or cuts (36, 36A) delineating between the removable portion (32, 32') of the removable cover leaf (21, 22) and the remaining non-removable portion (34A, 34').

11. A test device (10, 100, 300, 400) according to any preceding claim in which the at least one removable cover leaf (21, 22) comprising the moisture barrier layer comprises at least one of: a metal foil; a desiccant loaded plastic layer; a thermoplastic layer inwardly facing towards the test strip (20); a desiccant loaded thermoplastic layer inwardly facing towards the test strip; a laminate comprising a plastic outer layer, a central moisture barrier layer and an inwardly facing tie-in layer.

12. A test device (10, 100, 300, 400) according to any preceding claim in which the removable cover leaf (21, 22) is a first removable cover leaf having a removable portion (32, 32') extending over at least part of one of the major surfaces (28, 29) of the test strip so as to seal the test area; and,
in which a second-removable cover leaf (21, 22) is provided on the other major surface (28, 29) of the test strip (20) having a second removable portion (32, 32') extending over and attached to at least a part of said other major surface (28, 29) of the test strip (20) so as to seal the test area and having a second non-removable portion (34A, 34') attached to the separable distal portion (16) of the test strip (20).

13. A method of manufacturing a test device (10, 100, 300, 400) according to any preceding claim comprising one or more of the following steps:
a) forming a test strip (20) having first and second major surfaces (28, 29) and comprising:
i) a proximal portion (12) for bearing contacts (18);
ii) an intermediate portion (14) for receiving a sample comprising a test area comprising at least one test component;
iii) a separable distal portion (16);
b) applying a first removable cover leaf (21, 22) extending over at least part of one of the major surfaces (28, 29) of the test strip (20) so as to seal the test area;
c) attaching removably pre-selected regions of the removable portion (32, 32') of the first removable cover leaf to the major surface of the test strip to form a seal about the test area;
d) attaching non-removably a non-removable portion (34A, 34') of the first removable cover leaf (21, 22) to the separable distal portion (16) of the test strip (20);
e) attaching pre-selected regions of the removable portion (32, 32') of the first removable cover leaf to the test strip in a manner so that at least part or all of the removable portion (32, 32') of the removable cover leaf (21, 22) is not attached, or is not attached to any appreciable extent, to the intermediate portion (14), or to the test area, of the test strip (20).

14. A method according to claims 13 comprising providing cuts (30A) and/or lines of weakness (36, 36A) in the test strip (20) and/or in one or each cover leaf (21, 22) before and/or during and/or after assembly of the test device (10, 100, 300, 400).

15. A test device card comprising two or more test devices (10, 100, 300, 400) according to claim 1 to 12 or manufactured according to the method of claims 13 or 14 having frangible connections between test devices (10, 100, 300, 400), optionally
comprising a row of test devices (10, 100, 300, 400), optionally, comprising 2, 3, 4, 5, or 6 of the test devices (10, 100, 300, 400).

## Patentansprüche

1. Testvorrichtung (10, 100, 300, 400), die Folgendes umfasst:
einen Teststreifen (20), der eine erste und zweite Hauptfläche (28, 29) aufweist und Folgendes umfasst:
- einen proximalen Abschnitt (12) zum Tragen von Kontakten (18) für eine Messvorrichtung,
- einen Zwischenabschnitt (14) zum Aufnehmen einer Probe, der einen Testbereich auf der ersten Hauptfläche (28) umfasst, wobei der Testbereich mindestens eine Testkomponente umfasst;
- einen trennbaren distalen Abschnitt (16); der trennbar ist, um so in Bezug auf mindestens den Zwischenabschnitt (14) bewegbar zu sein;
und wobei die Testvorrichtung (10, 100, 300, 400) ferner Folgendes umfasst:
mindestens ein entfernbares Deckblatt (21, 22), das eine Feuchtigkeitsbarriereschicht umfasst;
wobei das mindestens eine entfernbare Deckblatt (21, 22) einen entfernbaren Abschnitt (32, 32') umfasst, der sich über mindestens einen Teil von einer der Hauptflächen (28, 29) des Teststreifens (20) erstreckt, um so den Testbereich gegen Eindringen von Feuchtigkeit abzudichten, und
das mindestens eine entfernbare Deckblatt (21, 22) ferner einen nicht entfernbaren Abschnitt (34A, 34') umfasst, der am trennbaren distalen Abschnitt (16) des Teststreifens (20) angebracht ist.

2. Testvorrichtung (10, 100, 300, 400) gemäß Anspruch 1, wobei sich der entfernbare Abschnitt (32, 32') des entfernbaren Deckblatts (21, 22) über die erste Hauptfläche (28) des Teststreifens (20) erstreckt, um den Testbereich in Bezug auf die erste Hauptfläche (28) abzudichten, oder wobei sich der entfernbare Abschnitt (32, 32') des entfernbaren Deckblatts (21, 22) über die zweite Hauptfläche (29) des Teststreifens erstreckt, um so den Testbereich in Bezug auf die zweite Hauptfläche (29) abzudichten.

3. Testvorrichtung (10, 100, 300, 400) gemäß Anspruch 1 oder 2, wobei der trennbare distale Abschnitt (16) des Teststreifens (20) in Bezug auf den Zwischenabschnitt (14) und/oder den proximalen Abschnitt (12) des Teststreifens (20) rotierbar ist
und/oder
wobei der trennbare distale Abschnitt (16) des Teststreifens (20) mit dem Zwischenabschnitt (14) und/oder proximalen Abschnitt (12) des Teststreifens (20) durch einen oder mehrere Gelenkabschnitte (30, 30') verbunden ist.

4. Testvorrichtung (10, 100, 300, 400) gemäß einem vorhergehenden Anspruch, wobei der trennbare distale Abschnitt (16) mindestens einen Arm umfasst, der entlang dem Zwischenabschnitt (14) des Teststreifens (20) liegt,
und/oder
wobei der trennbare distale Abschnitt (16) einen Rahmen (16) um den Zwischenabschnitt (14) des Teststreifens (20) bildet.

5. Testvorrichtung (10, 100, 300, 400) gemäß Anspruch 4, wobei der trennbare distale Abschnitt (16) U-förmig ist und zwei lateral beabstandete Arme, die entlang dem Zwischenabschnitt (14) liegen, und eine distale Querstrebe dazwischen aufweist, um den Rahmen (16) um den Zwischenabschnitt (14) zu bilden,
und/oder
wobei ein Gelenkabschnitt (30, 30') zwischen einem oder jedem Arm und dem proximalen Abschnitt (12) oder zwischen dem Rahmen (16) und dem proximalen Abschnitt (12) bereitgestellt ist.

6. Testvorrichtung (10, 100, 300, 400) gemäß einem vorhergehenden Anspruch, wobei die Größe und/oder Form der Peripherie des trennbaren distalen Abschnitts (16) gegenüber dem Zwischenabschnitt (14) der Größe und/oder Form der gegenüberliegenden Peripherie des Zwischenabschnitts (14) entsprechen/entspricht, wobei diese unmittelbar nebeneinander liegen.

7. Testvorrichtung (10, 100, 300, 400) gemäß einem vorhergehenden Anspruch, wobei der Zwischenabschnitt (14) eine schmalere Breite als der proximale Abschnitt (12) aufweist und einen Zwischenspitzenabschnitt (14) bildet, der eine Spitze umfasst,
und/oder
wobei der trennbare distale Abschnitt (16) des Teststreifens einen Rahmen (16) um den Zwischenabschnitt (14) des Teststreifens (20) bildet und sich der entfernbare Abschnitt (32, 32') des entfernbaren Deckblatts (21, 22) über mindestens den Zwischenabschnitt (14) erstreckt und der nicht entfernbare Abschnitt (34A, 34') des entfernbaren Deckblatts (21, 22) über den Rahmen (16) erstreckt.

8. Testvorrichtung (10, 100, 300, 400) gemäß einem vorhergehenden Anspruch, wobei sich der entfernbare Abschnitt (32, 32') des entfernbaren Deckblatts (21, 22) i) über mindestens einen Teil des oder den gesamten Zwischenabschnitt(s) (14) oder ii) über mindestens einen Teil des oder den gesamten Zwischenabschnitt (14) und über mindestens einen Teil des oder den gesamten proximalen Abschnitt(s) (12) erstreckt.

9. Testvorrichtung (10, 100, 300, 400) gemäß einem vorhergehenden Anspruch, wobei:
i) der entfernbare Abschnitt (32, 32') des mindestens einen entfernbaren Deckblatts (21, 22) vorselektierte am Teststreifen (20) angebrachte Regionen umfasst, die mindestens einen Teil des oder den gesamten Zwischenabschnitt(s) (14) umgeben oder mindestens einen Teil des oder den gesamten Testbereich(s) des Teststreifens (20) umgeben und
die am Teststreifen (20) angebrachten vorselektierten Regionen des entfernbaren Abschnitts (32, 32') auf eine Weise angebracht sind, dass mindestens ein Teil des oder der gesamte(n) entfernbare(n) Abschnitt(s) (32, 32') des entfernbaren Deckblatts (21, 22) am Zwischenbereich (14) nicht angebracht ist oder nicht in einem nennenswerten Maß angebracht ist oder am Testbereich des Teststreifens (20) nicht angebracht oder nicht in einem nennenswerten Maß angebracht ist,
oder
ii) wobei der nicht entfernbare Abschnitt (34A, 34') des mindestens einen entfernbaren Deckblatts (21, 22) am trennbaren distalen Abschnitt (16) des Teststreifens (20) permanent angebracht ist und der entfernbare Abschnitt (32, 32') mindestens am Zwischenabschnitt (14) des Teststreifens (20) abziehbar angebracht ist.

10. Testvorrichtung (10, 100, 300, 400) gemäß einem vorhergehenden Anspruch, wobei der Teststreifen (20) ein Substrat (50) umfasst und das Substrat (50) eine integrale Komponente ist und einen proximalen Abschnitt (12A), einen Zwischenabschnitt (14A) und einen trennbaren distalen Abschnitt (16A) umfasst
und/oder
wobei das Substrat (50) eine oder mehrere Schwächungslinien (30A) und/oder Schnitte (36, 36A) umfasst, welche zwischen dem Zwischenabschnitt (14) und dem trennbaren distalen Abschnitt (16) liegen,
und/oder
wobei das Substrat (50) eine oder mehrere Schwächungslinien (30A) umfasst, die zwischen dem proximalen Abschnitt (12) und dem trennbaren distalen Abschnitt (16) liegen,
und/oder
wobei das mindestens eine entfernbare Deckblatt (21, 22) eine oder mehrere Schwächungslinien (30A) und/oder Schnitte (36, 36A) umfasst, die zwischen dem entfernbaren Abschnitt (32, 32') des entfernbaren Deckblatts (21, 22) und dem restlichen nicht entfernbaren Abschnitt (34A, 34') liegen.

11. Testvorrichtung (10, 100, 300, 400) gemäß einem vorhergehenden Anspruch, wobei das mindestens eine entfernbare Deckblatt (21, 22), das die Feuchtigkeitsbarriereschicht umfasst, mindestens eines der Folgenden umfasst: eine Metallfolie; eine mit Trockenmittel beladene Kunststoffschicht; eine thermoplastische Schicht, die nach innen zum Teststreifen (20) gewandt ist; eine mit Trockenmittel beladene thermoplastische Schicht, die nach innen zum Teststreifen gewandt ist; ein Laminat, das eine Kunststoffaußenschicht, eine mittlere Feuchtigkeitsbarriereschicht und eine nach innen gewandte Verbundschicht umfasst.

12. Testvorrichtung (10, 100, 300, 400) gemäß einem vorhergehenden Anspruch, wobei das entfernbare Deckblatt (21, 22) ein erstes entfernbares Deckblatt ist, das einen entfernbaren Abschnitt (32, 32') aufweist, der sich mindestens über einen Teil von einer der Hauptflächen (28, 29) des Teststreifens erstreckt, um so den Testbereich abzudichten; und
wobei ein zweites entfernbares Deckblatt (21, 22) auf der anderen Hauptfläche (28, 29) des Teststreifens (20) bereitgestellt ist, das einen zweiten entfernbaren Abschnitt (32, 32') aufweist, der sich über mindestens einen Teil der anderen Hauptfläche (28, 29) des Teststreifens (20) erstreckt und an diesem angebracht ist, um so den Testbereich abzudichten, und einen zweiten nicht entfernbaren Abschnitt (34A, 34') aufweist, der am trennbaren distalen Abschnitt (16) des Teststreifens (20) angebracht ist.

13. Verfahren zum Herstellen einer Testvorrichtung (10, 100, 300, 400) gemäß einem vorhergehenden Anspruch, das einen oder mehrere der folgenden Schritte umfasst:
a) Bilden eines Teststreifens (20), der eine erste und zweite Hauptfläche (28, 29) aufweist und Folgendes umfasst:
i) einen proximalen Abschnitt (12) zum Tragen von Kontakten (18);
ii) einen Zwischenabschnitt (14) zum Aufnehmen einer Probe, der einen Testbereich umfasst, der mindestens eine Testkomponente umfasst;
iii) einen trennbaren distalen Abschnitt (16);
b) Aufbringen eines ersten entfernbaren Deckblatts (21, 22), das sich über mindestens einen Teil von einer der Hauptflächen (28, 29) des Teststreifens (20) erstreckt, um so den Testbereich abzudichten;
c) entfernbares Anbringen der vorselektierten Regionen des entfernbaren Abschnitts (32, 32') des ersten entfernbaren Deckblatts an der Hauptfläche des Teststreifens, um eine Dichtung um den Testbereich zu bilden;
d) nicht entfernbares Anbringen eines nicht entfernbaren Abschnitts (34A, 34') des ersten entfernbaren Deckblatts (21, 22) am trennbaren distalen Abschnitt (16) des Teststreifens (20);
e) Anbringen der vorselektierten Regionen des entfernbaren Abschnitts (32, 32') des ersten entfernbaren Deckblatts am Teststreifen auf eine Weise, dass mindestens ein Teil des oder der gesamte(n) entfernbare(n) Abschnitt(s) (32, 32') des entfernbaren Deckblatts (21, 22) am Zwischenbereich (14) nicht angebracht ist oder nicht in einem nennenswerten Maß angebracht ist oder am Testbereich des Teststreifens (20) nicht angebracht oder nicht in einem nennenswerten Maß angebracht ist.

14. Verfahren gemäß Anspruch 13, das das Bereitstellen von Schnitten (30A) und/oder Schwächungslinien (36, 36A) im Teststreifen (20) und/oder in einem oder jedem Deckblatt (21, 22) vor und/oder während und/oder nach der Montage der Testvorrichtung (10, 100, 300, 400) umfasst.

15. Testvorrichtungskarte, die zwei oder mehrere Testvorrichtungen (10, 100, 300, 400) gemäß Anspruch 1 bis 12 oder hergestellt gemäß dem Verfahren gemäß Anspruch 13 oder 14 umfasst, mit zerbrechlichen Verbindungen zwischen den Testverrichtungen (10, 100, 300, 400), fakultativ
eine Reihe von Testverrichtungen (10, 100, 300, 400) umfasst, fakultativ 2, 3, 4, 5 oder 6 der Testvorrichtungen (10, 100, 300, 400) umfasst.

## Revendications

1. Dispositif d'essai (10, 100, 300, 400) comprenant :
un bâtonnet diagnostique (20) possédant des première et seconde surfaces majeures (28, 29) et comprenant :
une partie proximale (12) pour le support de contacts (18) pour un dispositif de mesure,
une partie intermédiaire (14) pour la réception d'un échantillon comprenant une zone d'essai sur la première surface majeure (28), ladite zone d'essai comprenant au moins un composant d'essai ;
une partie distale apte à être séparée (16) ; apte à être séparée de façon à être mobile par rapport à la partie intermédiaire (14) au moins ;
et ledit dispositif d'essai (10, 100, 300, 400) comprenant en outre :
au moins une feuille de couverture amovible (21, 22) comprenant une couche barrière d'humidité ;
la au moins une feuille de couverture amovible (21, 22) comprenant une partie amovible (32, 32') s'étendant sur au moins une partie de l'une des surfaces majeures (28, 29) du bâtonnet diagnostique (20) de manière à sceller la zone d'essai d'une pénétration de l'humidité et,
la au moins une feuille de couverture amovible (21, 22) comprenant en outre une partie non amovible (34A, 34') fixée à la partie distale apte à être séparée (16) du bâtonnet diagnostique (20).

2. Dispositif d'essai (10, 100, 300, 400) selon la revendication 1, ladite partie amovible (32, 32') de la feuille de couverture amovible (21, 22) s'étendant sur la première surface majeure (28) du bâtonnet diagnostique (20) pour sceller la zone d'essai par rapport à la première surface majeure (28), ou ladite partie amovible (32, 32') de la feuille de couverture amovible (21, 22) s'étendant sur la seconde surface majeure (29) du bâtonnet diagnostique de manière à sceller la zone d'essai par rapport à la seconde surface majeure (29).

3. Dispositif d'essai (10, 100, 300, 400) selon la revendication 1 ou 2, ladite partie distale apte à être séparée (16) du bâtonnet diagnostique (20) pouvant pivoter par rapport à la partie intermédiaire (14) et/ou à la partie proximale (12) du bâtonnet diagnostique (20),
et/ou,
ladite partie distale apte à être séparée (16) du bâtonnet diagnostique (20) étant reliée à la partie intermédiaire (14) et/ou la partie proximale (12) du bâtonnet diagnostique (20) par une ou plusieurs parties charnières (30, 30').

4. Dispositif d'essai (10, 100, 300, 400) selon l'une quelconque des revendications précédentes, ladite partie distale apte à être séparée (16) comprenant au moins un bras se trouvant le long du côté de la partie intermédiaire (14) du bâtonnet d'essai (20),
et/ou,
ladite partie distale apte à être séparée (16) formant un cadre (16) autour de ladite partie intermédiaire (14) du bâtonnet diagnostique (20).

5. Dispositif d'essai (10, 100, 300, 400) selon la revendication 4, ladite partie distale apte à être séparée (16) étant en forme de U possédant deux bras espacés latéralement se trouvant le long des côtés de la partie intermédiaire (14) et une traverse distale entre ceux-ci pour former le cadre (16) autour de la partie intermédiaire (14),
et/ou,
une partie charnière (30, 30') étant disposée entre un ou chaque bras et la partie proximale (12), ou entre le cadre (16) et la partie proximale (12).

6. Dispositif d'essai (10, 100, 300, 400) selon l'une quelconque des revendications précédentes, ladite dimension et/ou ladite forme de la périphérie de la partie distale apte à être séparée (16) opposée à la partie intermédiaire (14) correspondant à la dimension et/ou la forme de la périphérie opposée de la partie intermédiaire (14), moyennant quoi celles-ci se trouvent à proximité immédiate l'une de l'autre.

7. Dispositif d'essai (10, 100, 300, 400) selon l'une quelconque des revendications précédentes, ladite partie intermédiaire (14) étant d'une largeur plus étroite que la partie proximale (12) et formant une partie pointe intermédiaire (14), comprenant une pointe,
et/ou,
ladite partie distale apte à être séparée (16) du bâtonnet diagnostique formant un cadre (16) autour de la partie intermédiaire (14) du bâtonnet diagnostique (20) et la partie amovible (32, 32') de la feuille de couverture amovible (21, 22) s'étendant sur au moins la partie intermédiaire (14) et la partie non amovible (34A, 34') de la feuille de couverture amovible (21, 22) s'étendant sur le cadre (16).

8. Dispositif d'essai (10, 100, 300, 400) selon l'une quelconque des revendications précédentes, ladite partie amovible (32, 32') de la feuille de couverture amovible (21, 22) s'étendant i) sur au moins une partie ou la totalité de la partie intermédiaire (14), ou ii) sur au moins une partie ou la totalité de la partie intermédiaire (14) et sur au moins une partie ou la totalité de la partie proximale (12).

9. Dispositif d'essai (10, 100, 300, 400) selon l'une quelconque des revendications précédentes:
i) ladite partie amovible (32, 32') de la au moins une feuille de couverture amovible (21, 22) comprenant des zones présélectionnées fixées au bâtonnet diagnostique (20) entourant au moins une partie ou la totalité de la partie intermédiaire (14), ou entourant au moins une partie ou la totalité de la zone d'essai du bâtonnet diagnostique (20) et,
lesdites zones présélectionnées de la partie amovible (32, 32'), fixées au bâtonnet diagnostique (20) étant fixées de manière qu'au moins un partie ou la totalité de la partie amovible (32, 32') de la feuille de couverture amovible (21, 22) ne soit pas fixée ou ne soit pas fixée de façon appréciable à la partie intermédiaire (14), ou ne soit pas fixée ou ne soit pas fixée de façon appréciable à la zone d'essai, du bâtonnet diagnostique (20),
ou,
ii) ladite partie non amovible (34A, 34') de la au moins une feuille de couverture amovible (21, 22) étant fixée en permanence à la partie distale apte à être séparée (16) du bâtonnet diagnostique (20) et la partie amovible (32, 32') étant fixée de manière détachable à la partie intermédiaire (14), au moins, du bâtonnet diagnostique (20).

10. Dispositif d'essai (10, 100, 300, 400) selon l'une des revendications précédentes, ledit bâtonnet diagnostique (20) comprenant un substrat (50) et ledit substrat (50) étant un composant intégré et comprenant une partie proximale (12A), une partie intermédiaire (14A) et une partie distale apte à être séparée (16A),
et/ou,
ledit substrat (50) comprenant une ou plusieurs lignes de faiblesse (30A) et/ou des coupures (36, 36A) délimitant la partie intermédiaire (14) de la partie distale apte à être séparée (16), et/ou,
ledit substrat (50) comprenant une ou plusieurs lignes de faiblesse (30A) délimitant la partie proximale (12) de la partie distale apte à être séparée (16),
et/ou,
ladite au moins une feuille de couverture amovible (21, 22) comprenant une ou plusieurs lignes de faiblesse (30A) et/ou des coupures (36, 36A) délimitant la partie amovible (32, 32') de la feuille de couverture amovible (21, 22) de la partie non amovible restante (34A, 34').

11. Dispositif d'essai (10, 100, 300, 400) selon l'une quelconque des revendications précédentes, ladite au moins une feuille de couverture amovible (21, 22) comprenant la couche barrière d'humidité comprenant au moins l'un de : une feuille métallique ; une couche plastique chargée de déshydratant ; une couche thermoplastique faisant face vers l'intérieur en direction du bâtonnet diagnostique (20) ; une couche thermoplastique chargée de déshydratant faisant face vers l'intérieur en direction du bâtonnet diagnostique ; un stratifié comprenant une couche extérieure en plastique, une couche barrière d'humidité centrale et une couche de raccord faisant face vers l'intérieur.

12. Dispositif d'essai (10, 100,300,400) selon l'une quelconque des revendications précédentes, ladite feuille de couverture amovible (21,22) étant une première feuille de couverture amovible comportant une partie amovible (32, 32') s'étendant sur au moins une partie de l'une des surfaces majeures (28, 29) du bâtonnet diagnostique de manière à sceller la zone d'essai ; et,
une seconde feuille de couverture amovible (21, 22) étant disposée sur l'autre surface majeure (28, 29) du bâtonnet diagnostique (20) possédant une seconde partie amovible (32, 32') s'étendant sur et étant fixé à au moins une partie de ladite autre surface majeure (28, 29) du bâtonnet diagnostique (20) de façon à sceller la zone d'essai et comportant une seconde partie non amovible (34A, 34') fixée à la partie distale apte à être séparée (16) du bâtonnet diagnostique (20).

13. Procédé de fabrication d'un dispositif d'essai (10, 100, 300, 400) selon l'une quelconque des revendications précédentes comprenant une ou plusieurs des étapes suivantes :
a) formation d'un bâtonnet diagnostique (20) comportant des première et seconde surfaces majeures (28, 29) et comprenant :
i) une partie proximale (12) pour le support de contacts (18) ;
ii) une partie intermédiaire (14) pour la réception d'un échantillon comprenant une zone d'essai comportant au moins un composant d'essai ;
iii) une partie distale apte à être séparée (16) ;
b) application d'une première feuille de couverture amovible (21, 22) s'étendant sur au moins une partie de l'une des surfaces majeures (28, 29) du bâtonnet diagnostique (20) de façon à sceller la zone d'essai ;
c) la fixation de manière amovible de zones présélectionnées de la partie amovible (32, 32') de la première feuille de couverture amovible à la surface majeure du bâtonnet diagnostique afin de former un joint autour la zone d'essai ;
d) fixation de manière non amovible d'une partie non amovible (34A, 34') de la première feuille de couverture amovible (21, 22) à la partie distale apte à être séparée (16) du bâtonnet diagnostique (20) ;
e) fixation de zones présélectionnées de la partie amovible (32, 32') de la première feuille de couverture amovible au bâtonnet diagnostique de manière qu'au moins une partie ou la totalité de la partie amovible (32, 32') de la feuille de couverture amovible (21, 22) ne soit pas fixée, ou ne soit pas fixée de façon appréciable, à la partie intermédiaire (14) ou à la zone d'essai, du bâtonnet diagnostique (20).

14. Procédé selon la revendication 13, comprenant la disposition de coupures (30A) et/ou de lignes de faiblesse (36, 36A) dans le bâtonnet diagnostique (20) et/ou dans une ou chaque feuille de couverture (21,22), avant et/ou pendant et/ou après l'assemblage du dispositif d'essai (10, 100, 300, 400).

15. Carte de dispositifs d'essai comprenant deux dispositifs d'essai (10, 100, 300, 400), ou plus, selon les revendications 1 à 12 ou fabriqués selon le procédé des revendications 13 ou 14, comportant des raccords pouvant être rompus entre les dispositifs d'essai (10, 100, 300, 400), comprenant éventuellement une rangée de dispositifs d'essai (10, 100, 300, 400), éventuellement, comprenant 2, 3, 4, 5 ou 6 des dispositifs d'essai (10, 100, 300, 400).
